(19) Europäisches Patentamt
European Patent Office
Office européen des brevets

(11) **EP 3 445 317 B1**

(12) **EUROPEAN PATENT SPECIFICATION**

(45) Date of publication and mention of the grant of the patent:
**22.11.2023 Bulletin 2023/47**

(21) Application number: **16788918.7**

(22) Date of filing: **21.10.2016**

(51) International Patent Classification (IPC):
***A61K 8/33*** $^{(2006.01)}$ ***A61K 8/34*** $^{(2006.01)}$
***A61K 8/37*** $^{(2006.01)}$ ***A61K 8/46*** $^{(2006.01)}$
***A61K 8/49*** $^{(2006.01)}$ ***A61Q 5/00*** $^{(2006.01)}$
***A61Q 5/02*** $^{(2006.01)}$ ***A61Q 13/00*** $^{(2006.01)}$
***A61Q 19/10*** $^{(2006.01)}$

(52) Cooperative Patent Classification (CPC):
**A61K 8/463; A61K 8/33; A61K 8/34; A61K 8/37; A61K 8/4926; A61K 8/4946; A61Q 5/006; A61Q 5/02; A61Q 13/00; A61Q 19/10**

(86) International application number:
**PCT/US2016/058123**

(87) International publication number:
**WO 2017/184199 (26.10.2017 Gazette 2017/43)**

(54) **DELIVERY OF SURFACTANT SOLUBLE AGENT**

VERABREICHUNG VON TENSIDLÖSLICHEN MITTELN

ADMINISTRATION D’AGENT SOLUBLE DANS UN TENSIOACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2016 US 201662326271 P**

(43) Date of publication of application:
**27.02.2019 Bulletin 2019/09**

(73) Proprietor: **The Procter & Gamble Company**
**Cincinnati, OH 45202 (US)**

(72) Inventors:
- **CHANG, Debora, W.**
  **Cincinnati, Ohio 45202 (US)**
- **JOHNSON, Eric, Scott**
  **Cincinnati, Ohio 45202 (US)**
- **GLENN, Robert, Wayne, Jr.**
  **Cincinnati, Ohio 45202 (US)**
- **THOMPSON, Todd, Ryan**
  **Cincinnati, Ohio 45202 (US)**
- **CARTER, Michelle, Lynn**
  **Cincinnati, Ohio 45202 (US)**
- **EDWARDS, Allison, Lynn**
  **Cincinnati, Ohio 45202 (US)**
- **EADS, Charles, David**
  **Cincinnati, Ohio 45202 (US)**
- **HERTENSTEIN, Stacy, Renee**
  **Cincinnati, Ohio 45202 (US)**
- **CORDER, Jennifer, Anne**
  **Cincinnati, Ohio 45202 (US)**
- **DIERSING, Steven, Louis**
  **Cincinnati, Ohio 45202 (US)**
- **LI, Jianjun, Justin**
  **Cincinnati, Ohio 45202 (US)**
- **PENG, Ruzhan**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**EP-A2- 0 338 850** **WO-A1-2004/056329**
**WO-A1-2016/172401** **WO-A1-2016/172409**
**WO-A2-2010/141683** **US-A1- 2006 057 075**
**US-A1- 2009 155 383** **US-A1- 2009 221 463**
**US-A1- 2013 280 202**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention is directed to delivery of a surfactant soluble agent from a personal care composition.

BACKGROUND OF THE INVENTION

For years, personal care products have been widely used to clean hair, scalp, face, and body, and a key signal that those components are clean is the fragrance left behind after washing. In general, personal care products are formulated with various benefit agents such as perfumes, conditioning agents, vitamins, antioxidants or anti-acne actives in combination with surfactants and aqueous systems that are intended to deposit those materials on the hair, scalp, face or body. The benefit agents can be surfactant insoluble materials such as dimethicone or petrolatum and/or surfactant soluble substances such as perfumes, salicylic acid, octopirox or climbazole. Many personal care products use cationic polymers with anionic surfactants to form coacervate which aid in the deposition of insoluble materials. However, generally coacervates do not impact soluble agent deposition as the soluble agents do not associate with the coacervates formed between the cationic polymers and anionic surfactants. Indeed it can prove difficult to deposit much more than 1-2% of the soluble agents present in personal care products while the remaining 98-99% of the soluble agents in the formulas are rinsed away. As many of the soluble agents can be relatively expensive, allowing >97% of the soluble agents to rinse away is equivalent to pouring money down the drain, and so there remains a need for a personal care product that can more efficiently deposit surfactant soluble agents.

WO 2010/141683 A2 is related to multiple product system for keratinic material suitable for cleansing mammalian hair. The system presents a regimen for providing improved conditioning benefits. The multiple product system for keratinic material comprises the steps of applying from 0.3 mL to t0.67 mL per 10g of hair of a hair cleaning composition comprising a dermatologically acceptable carrier, and from 3% to 40% of at least one surfactant selected from the group consisting of a branched and nonbranched versions of decyl and undecyl alkyl sulfates which are either ethoxylated or nonethoxylated; decyl alcohol modified lauryl sulfate; paraffin sulfonates with chain lengths ranging from C13 to C17 ; mixtures of linear and branched-chain alcohol sulfates with carbon chain lengths C12 to C17 which are ethoxylated or non-ethoxylated; sodium salts of branched, methyl-2-hydroxy-decyl ether sulfates, hydroxyethyl-2-dodecyl ether sulfates, hydroxyethyl-2-decyl ether sulfates; monoethoxylated lauryl alkyl sulfates; and mixtures thereof; and 0.05 wt% to 10 wt% of a silicone emulsion; and applying from 0.3 mL to 0.67 mL per 10 g of hair of a hair conditioning composition comprising: a cationic surfactant; a high melting point fatty compound; and an aqueous carrier; wherein the hair conditioning composition has a yield point of at least SPa,; wherein the application of the hair cleaning composition with or in series with the hair conditioning composition to keratinic material results in a silicone deposition of more than 350 ppm.

The association of many classes of surfactants into micellar aggregates is a well-known phenomenon. Micelles are often drawn as static structures of spherical aggregates, but in truth micelles are in dynamic equilibrium with individual surfactant molecules (monomers) that are constantly being exchanged between the bulk and the micelles. Additionally, the micelles themselves are continuously disintegrating and reassembling. There are two relaxation processes involved in micellar solutions. The first is a fast relaxation process referred to as $\tau_1$, which is associated with the quick exchange of monomers between micelles and the surrounding bulk phase. The second relaxation time, $\tau_2$, is attributed to the micelle formation and dissolution process (i.e., the lifetime of the micelle). Extensive experimental research on the kinetics of micellization by Shah and co-workers (Patist, A., Jha, B.K., Oh, S.G., and Shah, D.O., J. Surfactants Deterg. 2, 317, (1999); James-Smith, M.A., Shekhawat, D., and Shah, D.O., Tenside Surf. Det. 44, 142 (2007)) showed a strong correlation of $\tau_2$ with a number of detergency properties including oil solubilization in micellar solutions and droplet size in emulsions, as well as surfactant properties such as dynamic surface tension and micelle stability. Their research also showed a strong inverse correlation of $\tau_2$ with other properties such as foamability and concentration of sub-micellar aggregates. Specifically, they showed that a maximal $\tau_2$ and thus maximal micellar stability corresponded to both a maximal rate of oil solubilization and maximal amount of oil solubilized. Logic would therefore suggest that a cleansing composition with longer $\tau_2$, more stable micelles, and faster rate of solubilization would be preferred since such a system can clean better, more quickly solubilize larger quantities of oils or surfactant-soluble materials and should be more stable. Surprisingly however, it has been found that a composition with a surfactant system of shorter $\tau_2$, less stable micelles, and a slower rate of solubilization is preferred.

SUMMARY OF THE INVENTION

A personal care composition is provided and comprises from 14% to 40% of one or more surfactants, by weight of the total composition, wherein the one or more surfactants is an anionic surfactant selected from the group consisting of:

a)

$$R_1\ O(CH_2CHR_3O)_y\ SO_3M;$$

b)

$$CH_3\ (CH_2)_z\ CHR_2\ CH_2\ O\ (CH_2\ CHR_3O)_y\ SO_3M;$$

and

c) mixtures thereof,

where $R_1$ represents $CH_3\ (CH_2)_{10}$, $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is 1 when y is not zero (0), and M is a monovalent or divalent, positively-charged cation, wherein the total amount of one or more surfactants ranges from 14% to 40% by weight of the total composition;

from 0.1% to 10% of one or more surfactant soluble agents having a ClogP greater than 3.0, by weight of the total composition, preferably greater than 3.2; preferably greater than 3.4, as determined by the fragment approach of Hansch & Leo, in Comprehensive Medicinal Chemistry, Vol. 4, Hansch, et al., Eds., p. 295, Pergamon Press, 1990. The one or more surfactant soluble agents comprises a hair health active, wherein the hair health active is selected from the group consisting of isostearyl isostearate, histidine and mixtures thereof, wherein the total amount of one or more surfactant soluble agents having a ClogP greater than 3.0 ranges from 0.1% to 10% by weight of the total composition. When the personal care composition is diluted to 1.5% surfactant concentration has a ratio of surfactant diffusion coefficient to soluble agent diffusion coefficient greater than 1.2, preferably greater than 1.4, preferably greater than 1.6.

DETAILED DESCRIPTION OF EMBODIMENTS OF THE INVENTION

All percentages and ratios used herein are by weight of the total composition, unless otherwise designated. All measurements are understood to be made at ambient conditions, where "ambient conditions" means conditions at 25 °C, under one atmosphere of pressure, and at 50% relative humidity, unless otherwise designated. All numeric ranges are inclusive of narrower ranges; delineated upper and lower range limits are combinable to create further ranges not explicitly delineated.

The compositions of the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Apply" or "application," as used in reference to a composition, means to apply or spread the compositions of the present invention onto keratinous tissue such as the hair.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

While the specification concludes with claims particularly pointing out and distinctly claiming the invention, it is believed that the present invention will be better understood from the following description.

As used herein, the term "fluid" includes liquids and gels.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "Molecular weight" refers to the weight average molecular weight unless otherwise stated. Molecular weight is measured using industry standard method, gel permeation chromatography ("GPC").

As used herein, "personal care compositions" includes products such as shampoos, shower gels, liquid hand cleansers, hair colorants, facial cleansers, and other surfactant-based liquid compositions

As used herein, the terms "include," "includes," and "including," are meant to be non-limiting and are understood to mean "comprise," "comprises," and "comprising," respectively.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the hair care composition.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

While the $\tau_2$ and micelle stability of a cleansing composition surfactant system and the solubility of surfactant-soluble agents in that system are important, of equal importance are the surfactant micelle stability and solubility and rate of solubilization of the agents in the system after dilution, such as when the cleansing composition is applied to the head, face or body during use. One way of understanding the solubility and associations of the soluble agent in the surfactant system upon dilution is to measure by NMR the diffusion coefficients of the surfactant and the surfactant-soluble agents in a diluted sample. If the diffusion coefficients of the surfactant and the agent are similar such that the ratio of the two coefficients is close to 1.0, one is able to infer that the surfactant-soluble agent is within or closely associated with the surfactant micelles. However if the diffusion coefficients of the surfactant and the agent are very different such that the ratio of the two coefficients is significantly more or less than 1.0, then one is able to infer that the surfactant-soluble agent is not within or associated with the surfactant micelles. This in turn implies that the surfactant-soluble agent is less soluble in the diluted surfactant of the latter case.

It has been found that a soluble agent containing cleansing composition, wherein the soluble agent has a ClogP greater than 3.0, which when diluted to 1.5% surfactant concentration, has a ratio of surfactant diffusion coefficient to soluble agent diffusion coefficient greater than 1.2, can deposit that soluble agent with ~1.4X or greater efficiency than a soluble agent containing composition whose ratio of diffusion coefficients is close to 1.0.

In an embodiment of the present invention, when the personal care composition is diluted to 1.5% surfactant concentration has a ratio of surfactant diffusion coefficient to soluble agent diffusion greater than 1.4. In a further embodiment of the invention, when the personal care composition is diluted to 1.5% surfactant concentration has a ratio of surfactant diffusion coefficient to soluble agent diffusion greater than 1.6.

In an embodiment of the present invention, it has been found that a soluble agent containing cleansing composition, wherein the soluble agent has a ClogP greater than 3.0, which when diluted to 0.8% surfactant concentration, has a ratio of surfactant diffusion coefficient to soluble agent diffusion coefficient greater than 1.2. In a further embodiment of the present invention, it has been found that a soluble agent containing cleansing composition, wherein the soluble agent has a ClogP greater than 3.0, which when diluted to 1.0% surfactant concentration, has a ratio of surfactant diffusion coefficient to soluble agent diffusion coefficient greater than 1.2. In yet a further embodiment of the present invention, it has been found that a soluble agent containing cleansing composition, wherein the soluble agent has a ClogP greater than 3.0, which when diluted to 1.3% surfactant concentration, has a ratio of surfactant diffusion coefficient to soluble agent diffusion coefficient greater than 1.2. In a further embodiment of the present invention, it has been found that a soluble agent containing cleansing composition, wherein the soluble agent has a ClogP greater than 3.0, which when diluted to 1.5% surfactant concentration, has a ratio of surfactant diffusion coefficient to soluble agent diffusion coefficient greater than 1.2. In a further embodiment of the present invention, it has been found that a soluble agent containing cleansing composition, wherein the soluble agent has a ClogP greater than 3.0, which when diluted to 1.8% surfactant concentration, has a ratio of surfactant diffusion coefficient to soluble agent diffusion coefficient greater than 1.2. In yet another embodiment of the present invention, it has been found that a soluble agent containing cleansing composition, wherein the soluble agent has a ClogP greater than 3.0, which when diluted to 2.2% surfactant concentration, has a ratio of surfactant diffusion coefficient to soluble agent diffusion coefficient greater than 1.2

SURFACTANT SOLUBLE ACTIVE AGENT

The surfactant soluble active agent of the present invention is normally insoluble or only slightly soluble within water, but can be solubilized within aqueous surfactant solutions, in alcohol or in water/alcoholic mixtures. The surfactant soluble active agent may comprise vitamins, perfume oils, UV absorbers, anti-dandruff actives, scalp benefit agents, skin actives, internal hair actives, antimicrobials, antibacterial actives, radical scavengers, skin lightening agents, chelators, antioxidants, proteins, amino acids, peptides, flavonoids, alpha-hydroxy acids, anti-acne actives, antiinflammatories, and is included in an amount of from 0.1% to 10%, or from 0.2% to 5%, by weight of the composition. The surfactant soluble active agents of the present invention may have molecular weights ranging from 100 g/mol to 700 g/mol, or from 125 g/mol to 600 g/mol, or from 150 g/mol to 500 g/mol.

Surfactant soluble active agents are defined as materials which are insoluble or only slightly soluble in water but soluble at a concentration of 0.1% or higher in an aqueous solution of 10% sodium laureth-1 sulfate. A conventional method may be used to determine solubility. Such method may include a method wherein solubility of a material of interest can be determined by first visually assessing that the material containing the sodium laureth-1 sulfate mixture is homogeneous, followed by filling a glass jar with the material containing sodium laureth-1 sulfate mixture, then placing a Class 2 standard red laser pointer such as the Quartet Class 2 standard laser pointer (model MP-1202Q) against the side of the jar and shining the laser through the jar. If the material is soluble in the sodium laureth-1 solution the laser light will not be scattered, resulting in only an observable red dot appearing on the side of the jar opposite the laser pointer and no visible red laser beam will be observed passing through the solution.

Indeed the solubility of a material in water, surfactant solution or oil is primarily governed by the degree of hydrophilicity or conversely the degree of hydrophobicity of a material. As is conventional in the art, the degree of hydrophobicity of a material can be correlated with its octanol/water partitioning coefficient P. The octanol/water partitioning coefficient of a material is the ratio between its equilibrium concentration in octanol and in water. A material with a greater partitioning coefficient P is more hydrophobic and thus more oil soluble. Conversely, a material with a smaller partitioning coefficient P is more hydrophilic and thus more water soluble. The surfactant soluble materials of the present invention have a partitioning coefficient P that is less hydrophilic and more hydrophobic. Since the partitioning coefficients of materials can have high values, they are more conveniently given in the form of their logarithm to the base 10, logP. In particular embodiments, the logP values are more conveniently computed by the Consensus algorithm implemented in ACD/Percepta version 14.02 by Advanced Chemistry Development, Inc. (ACD/Labs, Toronto, Canada). The "calculated logP" (ClogP) is determined by the fragment approach of Hansch & Leo (cf., Leo, in Comprehensive Medicinal Chemistry, Vol. 4, Hansch, etal., Eds., p. 295, Pergamon Press, 1990). The fragment approach is based on the chemical structure of each material, and takes into account the numbers and types of atoms, the atom connectivity, and chemical bonding. The ClogP values, which are the most reliable and widely used estimates for this physicochemical property, are preferably used instead of the experimental logP values.

The surfactant soluble active agent or combination of agents have a ClogP greater than 3.0, preferably greater than 3.2, more preferably greater than 3.4. In this case, a ClogP greater than 3.0 denotes that the number average ClogP of all the soluble agent ingredients is greater than 3.0.

The surfactant soluble active agent comprises a hair health active which is one material or a mixture of materials, that is selected from isostearyl isostearate, histidine, and mixtures thereof.

A. DETERSIVE SURFACTANT

The personal care composition may comprise greater than 14% by weight of a surfactant system which provides cleaning performance to the composition, in an embodiment greater than 20% by weight of a surfactant system which provides cleaning performance to the composition. The surfactant system comprises an anionic surfactant and/or a combination of anionic surfactants and/or a combination of anionic surfactants and co-surfactants selected from the group consisting of amphoteric, zwitterionic, nonionic and mixtures thereof. Various examples and descriptions of detersive surfactants are set forth in U.S. Patent No. 8,440,605; U.S. Patent Application Publication No. 2009/155383; and U.S. Patent Application Publication No. 2009/0221463.

The personal care composition comprises from 14% to 40%, from 15% to 36%, from 18% to 32%, and/or from 20% to 28% by weight of one or more surfactants.

The composition of the present invention includes from 14% to 40% by weight of the total composition of anionic surfactants selected from the group consisting of:

a)

$$R_1\ O(CH_2CHR_3O)_y\ SO_3M;$$

b)

$$CH_3 (CH_2)_z CHR_2 CH_2 O (CH_2 CHR_3O)_y SO_3M;$$

and

c) mixtures thereof,

where $R_1$ represents $CH_3 (CH_2)_{10}$ , $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is 1 when y is not zero (0), and M is a monovalent or divalent, positively-charged cation.

The personal care composition may comprise a co-surfactant. The co-surfactant can be selected from the group consisting of amphoteric surfactant, zwitterionic surfactant, non-ionic surfactant and mixtures thereof. The co-surfactant can include, but is not limited to, lauramidopropyl betaine, cocoamidopropyl betaine, lauryl hydroxysultaine, sodium lauroamphoacetate, disodium cocoamphodiacetate, cocamide monoethanolamide and mixtures thereof.

The personal care composition may further comprise from 0.25% to 15%, from 2% to 14%, from 3% to 13% by weight of one or more amphoteric, zwitterionic, nonionic co-surfactants, or a mixture thereof.

Suitable amphoteric or zwitterionic surfactants for use in the personal care composition herein include those which are known for use in shampoo or other personal care cleansing. Non limiting examples of suitable zwitterionic or amphoteric surfactants are described in U.S. Patent Nos. 5,104,646 and 5,106,609.

Amphoteric co-surfactants suitable for use in the composition include those surfactants described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Suitable amphoteric surfactant include, but are not limited to, those selected from the group consisting of: sodium cocaminopropionate, sodium cocaminodipropionate, sodium cocoamphoacetate, sodium cocoamphodiacetate, sodium cocoamphohydroxypropylsulfonate, sodium cocoamphopropionate, sodium cornamphopropionate, sodium lauraminopropionate, sodium lauroamphoacetate, sodium lauroamphodiacetate, sodium lauroamphohydroxypropylsulfonate, sodium lauroamphopropionate, sodium cornamphopropionate, sodium lauriminodipropionate, ammonium cocaminopropionate, ammonium cocaminodipropionate, ammonium cocoamphoacetate, ammonium cocoamphodiacetate, ammonium cocoamphohydroxypropylsulfonate, ammonium cocoamphopropionate, ammonium cornamphopropionate, ammonium lauraminopropionate, ammonium lauroamphoacetate, ammonium lauroamphodiacetate, ammonium lauroamphohydroxypropylsulfonate, ammonium lauroamphopropionate, ammonium cornamphopropionate, ammonium lauriminodipropionate, triethanolamine cocaminopropionate, triethanolamine cocaminodipropionate, triethanolamine cocoamphoacetate, triethanolamine cocoamphohydroxypropylsulfonate, triethanolamine cocoamphopropionate, triethanolamine cornamphopropionate, triethanolamine lauraminopropionate, triethanolamine lauroamphoacetate, triethanolamine lauroamphohydroxypropylsulfonate, triethanolamine lauroamphopropionate, triethanolamine cornamphopropionate, triethanolamine lauriminodipropionate, cocoamphodipropionic acid, disodium caproamphodiacetate, disodium caproamphoadipropionate, disodium capryloamphodiacetate, disodium capryloamphodipriopionate, disodium cocoamphocarboxyethylhydroxypropylsulfonate, disodium cocoamphodiacetate, disodium cocoamphodipropionate, disodium dicarboxyethylcocopropylenediamine, disodium laureth-5 carboxyamphodiacetate, disodium lauriminodipropionate, disodium lauroamphodiacetate, disodium lauroamphodipropionate, disodium oleoamphodipropionate, disodium PPG-2-isodecethyl-7 carboxyamphodiacetate, lauraminopropionic acid, lauroamphodipropionic acid, lauryl aminopropylglycine, lauryl diethylenediaminoglycine, and mixtures thereof

The composition may comprises a zwitterionic co-surfactant, wherein the zwitterionic surfactant is a derivative of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. The zwitterionic surfactant can be selected from the group consisting of: cocamidoethyl betaine, cocamidopropylamine oxide, cocamidopropyl betaine, cocamidopropyl dimethylaminohydroxypropyl hydrolyzed collagen, cocamidopropyldimonium hydroxypropyl hydrolyzed collagen, cocamidopropyl hydroxysultaine, cocobetaineamido amphopropionate, coco-betaine, coco-hydroxysultaine, coco/oleamidopropyl betaine, coco-sultaine, lauramidopropyl betaine, lauryl betaine, lauryl hydroxysultaine, lauryl sultaine, and mixtures thereof.

Suitable nonionic surfactants for use in the present invention include those described in McCutcheion's Detergents and Emulsifiers, North American edition (1986), Allured Publishing Corp., and McCutcheion's Functional Materials, North American edition (1992). Suitable nonionic surfactants for use in the personal care compositions of the present invention include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl

glycosides, alkyl polyglucosides, alkylamine oxides, and polyoxyethylenated silicones.

The co-surfactant can be a non-ionic surfactant selected from the alkanolamides group including: Cocamide, Cocamide Methyl MEA, Cocamide DEA, Cocamide MEA, Cocamide MIPA, Lauramide DEA, Lauramide MEA, Lauramide MIPA, Myristamide DEA, Myristamide MEA, PEG-20 Cocamide MEA, PEG-2 Cocamide, PEG-3 Cocamide, PEG-4 Cocamide, PEG-5 Cocamide, PEG-6 Cocamide, PEG-7 Cocamide, PEG-3 Lauramide, PEG-5 Lauramide, PEG-3 Oleamide, PPG-2 Cocamide, PPG-2 Hydroxyethyl Cocamide, PPG-2 Hydroxyethyl Isostearamide and mixtures thereof.

Representative polyoxyethylenated alcohols include alkyl chains ranging in the C9-C16 range and having from 1 to 110 alkoxy groups including, but not limited to, laureth-3, laureth-23, ceteth-10, steareth-10, steareth-100, beheneth-10, and commercially available from Shell Chemicals, Houston, Texas under the trade names Neodol® 91, Neodol® 23, Neodol® 25, Neodol® 45, Neodol® 135, Neodo®l 67, Neodol® PC 100, Neodol® PC 200, Neodol® PC 600, and mixtures thereof.

Also available commercially are the polyoxyethylene fatty ethers available commercially under the Brij® trade name from Uniqema, Wilmington, Delaware, including, but not limited to, Brij® 30, Brij® 35, Brij® 52, Brij® 56, Brij® 58, Brij® 72, Brij® 76, Brij® 78, Brij® 93, Brij® 97, Brij® 98, Brij® 721 and mixtures thereof.

Suitable alkyl glycosides and alkyl polyglucosides can be represented by the formula (S)n-OR wherein S is a sugar moiety such as glucose, fructose, mannose, galactose, and the like; n is an integer of from 1 to 1000, and R is a C8-C30 alkyl group. Examples of long chain alcohols from which the alkyl group can be derived include decyl alcohol, lauryl alcohol, myristyl alcohol, cetyl alcohol, stearyl alcohol, oleyl alcohol, and the like. Examples of these surfactants include alkyl polyglucosides wherein S is a glucose moiety, R is a C8-20 alkyl group, and n is an integer of from 1 to 9. Commercially available examples of these surfactants include decyl polyglucoside and lauryl polyglucoside available under trade names APG® 325 CS, APG® 600 CS and APG® 625 CS) from Cognis, Ambler, Pa. Also useful herein are sucrose ester surfactants such as sucrose cocoate and sucrose laurate and alkyl polyglucosides available under trade names Triton™ BG-10 and Triton™ CG-110 from The Dow Chemical Company, Houston, Tx.

Other nonionic surfactants suitable for use in the present invention are glyceryl esters and polyglyceryl esters, including but not limited to, glyceryl monoesters, glyceryl monoesters of C12-22 saturated, unsaturated and branched chain fatty acids such as glyceryl oleate, glyceryl monostearate, glyceryl monopalmitate, glyceryl monobehenate, and mixtures thereof, and polyglyceryl esters of C12-22 saturated, unsaturated and branched chain fatty acids, such as polyglyceryl-4 isostearate, polyglyceryl-3 oleate, polyglyceryl-2- sesquioleate, triglyceryl diisostearate, diglyceryl monooleate, tetraglyceryl monooleate, and mixtures thereof.

Also useful herein as nonionic surfactants are sorbitan esters. Sorbitan esters of C12-22 saturated, unsaturated, and branched chain fatty acids are useful herein. These sorbitan esters usually comprise mixtures of mono-, di-, tri-, etc. esters. Representative examples of suitable sorbitan esters include sorbitan monolaurate (SPAN® 20), sorbitan monopalmitate (SPAN® 40), sorbitan monostearate (SPAN® 60), sorbitan tristearate (SPAN® 65), sorbitan monooleate (SPAN® 80), sorbitan trioleate (SPAN® 85), and sorbitan isostearate.

Also suitable for use herein are alkoxylated derivatives of sorbitan esters including, but not limited to, polyoxyethylene (20) sorbitan monolaurate (Tween® 20), polyoxyethylene (20) sorbitan monopalmitate (Tween® 40), polyoxyethylene (20) sorbitan monostearate (Tween® 60), polyoxyethylene (20) sorbitan monooleate (Tween® 80), polyoxyethylene (4) sorbitan monolaurate (Tween® 21), polyoxyethylene (4) sorbitan monostearate (Tween® 61), polyoxyethylene (5) sorbitan monooleate (Tween® 81), and mixtures thereof, all available from Uniqema.

Also suitable for use herein are alkylphenol ethoxylates including, but not limited to, nonylphenol ethoxylates (Tergitol™ NP-4, NP-6, NP-7, NP-8, NP-9, NP-10, NP-11, NP-12, NP-13, NP-15, NP-30, NP-40, NP-50, NP-55, NP-70 available from The Dow Chemical Company, Houston, Tx.) and octylphenol ethoxylates (Triton™ X-15, X-35, X-45, X-114, X-100, X-102, X-165, X-305, X-405, X-705 available from The Dow Chemical Company, Houston, Tx).

Also suitable for use herein are tertiary alkylamine oxides including lauramine oxide and cocamine oxide.

Non limiting examples of other anionic, zwitterionic, amphoteric, and non-ionic additional surfactants suitable for use in the personal care composition are described in McCutcheon's, Emulsifiers and Detergents, 1989 Annual, published by M. C. Publishing Co., and U.S. Patent Nos. 3,929,678, 2,658,072; 2,438,091; 2,528,378.

Suitable surfactant combinations comprise an average weight % of alkyl branching of from 0.5% to 30%, alternatively from 1% to 25%, alternatively from 2% to 20%. The surfactant combination can have a cumulative average weight % of C8 to C12 alkyl chain lengths of from 7.5% to 25%, alternatively from 10% to 22.5%, alternatively from 10% to 20%.The surfactant combination can have an average C8-C12 / C13-C18 alkyl chain ratio from 3 to 200, alternatively from 25 to 175.5, alternatively from 50 to 150, alternatively from 75 to 125.

B. CATIONIC POLYMERS

The personal care composition also comprises a cationic polymer. These cationic polymers can include at least one of (a) a cationic guar polymer, (b) a cationic non-guar galactomannan polymer, (c) a cationic tapioca polymer, (d) a cationic copolymer of acrylamide monomers and cationic monomers, and/or (e) a synthetic, non-crosslinked, cationic

polymer, which may or may not form lyotropic liquid crystals upon combination with the detersive surfactant (f) a cationic cellulose polymer. Additionally, the cationic polymer can be a mixture of cationic polymers.

The personal care composition may comprise a cationic guar polymer, which is a cationically substituted galactomannan (guar) gum derivatives. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of β(1-4) glycosidic linkages. The galactose branching arises by way of an α(1-6) linkage. Cationic derivatives of the guar gums are obtained by reaction between the hydroxyl groups of the polygalactomannan and reactive quaternary ammonium compounds. The degree of substitution of the cationic groups onto the guar structure should be sufficient to provide the requisite cationic charge density described above.

According to one embodiment, the cationic polymer, including but not limited to a cationic guar polymer, has a weight average Molecular weight of less than 1.5 million g/mol, or from 150 thousand to 1.5 million g/mol, or from 200 thousand to 1.5 million g/mol, or from 300 thousand to 1.2 million g/mol, or from 750,000 thousand to 1 million g/mol. In one embodiment, the cationic guar polymer has a charge density of from 0.2 to 2.2 meq/g, or from 0.3 to 2.0 meq/g, or from 0.4 to 1.8 meq/g; or from 0.5 meq/g to 1.7 meq/g.

According to one embodiment, the cationic guar polymer has a weight average Molecular weight of less than 1.5 million g/mol, and has a charge density of from 0.1 meq/g to 2.5 meq/g. In an embodiment, the cationic guar polymer has a weight average molecular weight of less than 900 thousand g/mol, or from 150 thousand to 800 thousand g/mol, or from 200 thousand to 700 thousand g/mol, or from 300 thousand to 700 thousand g/mol, or from 400 thousand to 600 thousand g/mol. from 150 thousand to 800 thousand g/mol, or from 200 thousand to 700 thousand g/mol, or from 300 thousand to 700 thousand g/mol, or from 400 thousand to 600 thousand g/mol. In one embodiment, the cationic guar polymer has a charge density of from 0.2 to 2.2 meq/g, or from 0.3 to 2.0 meq/g, or from 0.4 to 1.8 meq/g; or from 0.5 meq/g to 1.5 meq/g.

The personal care composition can comprise from 0.05% to less than 1%, from 0.05% to 0.9%, from 0.1% to 0.8%, or from 0.2% to 0.7% of cationic polymer (a), by total weight of the composition.

The cationic guar polymer may be formed from quaternary ammonium compounds. In an embodiment, the quaternary ammonium compounds for forming the cationic guar polymer conform to the general formula 1:

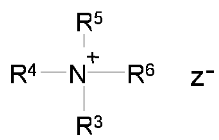

wherein where $R^3$, $R^4$ and $R^5$ are methyl or ethyl groups; $R^6$ is either an epoxyalkyl group of the general formula 2:

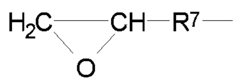

or $R^6$ is a halohydrin group of the general formula 3:

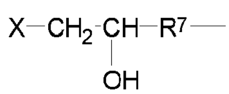

wherein $R^7$ is a $C_1$ to $C_3$ alkylene; X is chlorine or bromine, and Z is an anion such as Cl-, Br-, I- or $HSO_4$-.

In an embodiment, the cationic guar polymer conforms to the general formula 4:

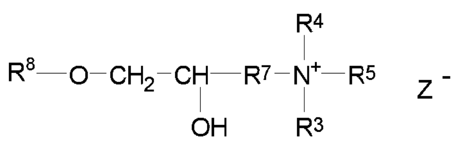

wherein $R^8$ is guar gum; and wherein $R^4$, $R^5$, $R^6$ and $R^7$ are as defined above; and wherein Z is a halogen. In an embodiment, the cationic guar polymer conforms to Formula 5:

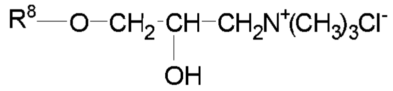

Suitable cationic guar polymers include cationic guar gum derivatives, such as guar hydroxypropyltrimonium chloride. In an embodiment, the cationic guar polymer is a guar hydroxypropyltrimonium chloride. Specific examples of guar hydroxypropyltrimonium chlorides include the Jaguar® series commercially available from Solvay, for example Jaguar® C-500, commercially available from Solvay. Jaguar® C-500 has a charge density of 0.8 meq/g and a molecular weight of 500,000 g/mol. Other suitable guar hydroxypropyltrimonium chloride are: guar hydroxypropyltrimonium chloride which has a charge density of 1.1 meq/g and a molecular weight of 500,000 g/mol is available from ASI, a charge density of 1.5 meq/g and a molecular weight of 500,000 g/mole is available from ASI. Other suitable guar hydroxypropyltrimonium chloride are: Hi-Care 1000, which has a charge density of 0.7 meq/g and a Molecular weight of 600,000 g/mole and is available from Solvay; N-Hance 3269 and N-Hance 3270, which have a charge density of 0.7 meq/g and a molecular weight of 425,000 g/mol and are available from ASI; N-Hance 3196, which has a charge density of 0.8 meq/g and a molecular weight of 1,100,000 g/ mol and is available from ASI. AquaCat CG518 has a charge density of 0.9 meq/g and a Molecular weight of 50,000 g/mol and is available from ASI. BF-13, which is a borate (boron) free guar of charge density of 1.1 meq/g and molecular weight of 800,000 and BF-17, which is a borate (boron) free guar of charge density of 1.7 meq/g and M. Wt. of 800,000 both available from ASI.

The personal care compositions of the present invention may comprise a galactomannan polymer derivative having a mannose to galactose ratio of greater than 2:1 on a monomer to monomer basis, the galactomannan polymer derivative selected from the group consisting of a cationic galactomannan polymer derivative and an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers are present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of β (1-4) glycosidic linkages. The galactose branching arises by way of an α (1-6) linkage. The ratio of mannose monomers to galactose monomers varies according to the species of the plant and also is affected by climate. Non Guar Galactomannan polymer derivatives of the present invention have a ratio of mannose to galactose of greater than 2:1 on a monomer to monomer basis. Suitable ratios of mannose to galactose can be greater than 3:1, and the ratio of mannose to galactose can be greater than 4:1. Analysis of mannose to galactose ratios is well known in the art and is typically based on the measurement of the galactose content.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose/1 part galactose), Locust bean or Carob (4 parts mannose/1 part galactose), and Cassia gum (5 parts mannose/1 part galactose).

In one embodiment of the invention, the non-guar galactomannan polymer derivatives have a M. Wt. from 1,000 to 10,000,000, and/or from 5,000 to 3,000,000.

The personal care compositions of the invention can also include galactomannan polymer derivatives which have a cationic charge density from 0.5 meq/g to 7 meq/g. In one embodiment of the present invention, the galactomannan polymer derivatives have a cationic charge density from 1 meq/g to 5 meq/g. The degree of substitution of the cationic groups onto the galactomannan structure should be sufficient to provide the requisite cationic charge density.

The galactomannan polymer derivative can be a cationic derivative of the non-guar galactomannan polymer, which is obtained by reaction between the hydroxyl groups of the polygalactomannan polymer and reactive quaternary ammonium compounds. Suitable quaternary ammonium compounds for use in forming the cationic galactomannan polymer derivatives include those conforming to the general formulas 1-5, as defined above.

Cationic non-guar galactomannan polymer derivatives formed from the reagents described above are represented by the general formula 6:

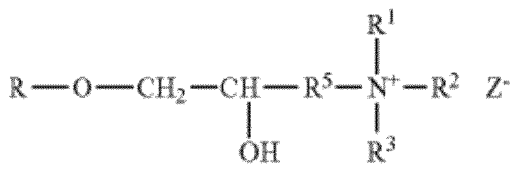

wherein R is the gum. The cationic galactomannan derivative can be a gum hydroxypropyltrimethylammonium chloride, which can be more specifically represented by the general formula 7:

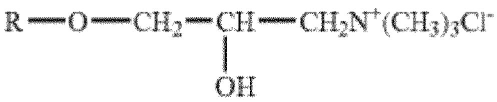

Alternatively the galactomannan polymer derivative can be an amphoteric galactomannan polymer derivative having a net positive charge, obtained when the cationic galactomannan polymer derivative further comprises an anionic group.

The cationic non-guar galactomannan can have a ratio of mannose to galactose is greater than 4:1, a molecular weight of 1,000 g/mol to 10,000,000 g/mol, and/or from 50,000 g/mol to 1,000,000 g/mol, and/or from 100,000 g/mol to 900,000 g/mol, and/or from 150,000 g/mol to 400,000 g/mol and a cationic charge density from 1 meq/g to 5 meq/g, and/or from 2 meq/ g to 4 meq/ g and can be derived from a cassia plant.

The personal care compositions can comprise at least 0.05% of a galactomannan polymer derivative by weight of the composition, alternatively from 0.05% to 2%, by weight of the composition, of a galactomannan polymer derivative.

The personal care compositions can comprise water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The personal care compositions can comprise cationically modified starch polymers at a range of 0.01% to 10%, and/or from 0.05% to 5%, by weight of the composition.

The cationically modified starch polymers disclosed herein have a percent of bound nitrogen of from 0.5% to 4%.

The cationically modified starch polymers for use in the personal care compositions can have a molecular weight 850,000 g/mol to 1,500,000 g/mol and/or from 900,000 g/mol to 1,500,000 g/mol.

The personal care compositions can include cationically modified starch polymers which have a charge density of from 0.2 meq/g to 5 meq/g, and/or from 0.2 meq/g to 2 meq/g. The chemical modification to obtain such a charge density includes, but is not limited to, the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O. B., Ed., CRC Press, Inc., Boca Raton, Fla. 1986, pp 113-125. The cationic groups may be added to the starch prior to degradation to a smaller molecular weight or the cationic groups may be added after such modification.

The cationically modified starch polymers generally have a degree of substitution of a cationic group from 0.2 to 2.5. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (" .sup.1H NMR") methods well known in the art. Suitable .sup.1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The source of starch before chemical modification can be chosen from a variety of sources such as tubers, legumes, cereal, and grains. Non-limiting examples of this source starch may include corn starch, wheat starch, rice starch, waxy corn starch, oat starch, cassava starch, waxy barley, waxy rice starch, glutenous rice starch, sweet rice starch, amioca, potato starch, tapioca starch, oat starch, sago starch, sweet rice, or mixtures thereof.

The cationically modified starch polymers can be selected from degraded cationic maize starch, cationic tapioca, cationic potato starch, and mixtures thereof. Alternatively, the cationically modified starch polymers are cationic corn starch and cationic tapioca.

The starch, prior to degradation or after modification to a smaller molecular weight, may comprise one or more additional modifications. For example, these modifications may include crosslinking, stabilization reactions, phosphorylations, and hydrolyzations. Stabilization reactions may include alkylation and esterification.

The cationically modified starch polymers may be incorporated into the composition in the form of hydrolyzed

starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

An optimal form of the starch is one which is readily soluble in water and forms a substantially clear (% Transmittance of 80 at 600 nm) solution in water. The transparency of the composition is measured by Ultra-Violet/Visible (UV/VIS) spectrophotometry, which determines the absorption or transmission of UV/VIS light by a sample, using a Gretag Macbeth Colorimeter Color i 5 according to the related instructions. A light wavelength of 600 nm has been shown to be adequate for characterizing the degree of clarity of cosmetic compositions.

Suitable cationically modified starch for use in personal care compositions are available from known starch suppliers. Also suitable for use in personal care compositions are nonionic modified starch that can be further derivatized to a cationically modified starch as is known in the art. Other suitable modified starch starting materials may be quaternized, as is known in the art, to produce the cationically modified starch polymer suitable for use in personal care compositions.

Starch Degradation Procedure: a starch slurry can be prepared by mixing granular starch in water. The temperature is raised to 35°C. An aqueous solution of potassium permanganate is then added at a concentration of 50 ppm based on starch. The pH is raised to 11.5 with sodium hydroxide and the slurry is stirred sufficiently to prevent settling of the starch. Then, a 30% solution of hydrogen peroxide diluted in water is added to a level of 1% of peroxide based on starch. The pH of 11.5 is then restored by adding additional sodium hydroxide. The reaction is completed over a 1 to 20 hour period. The mixture is then neutralized with dilute hydrochloric acid. The degraded starch is recovered by filtration followed by washing and drying.

The personal care composition can comprise a cationic copolymer of an acrylamide monomer and a cationic monomer, wherein the copolymer has a charge density of from 1.0 meq/g to 3.0 meq/g. The cationic copolymer can be a synthetic cationic copolymer of acrylamide monomers and cationic monomers.

The cationic copolymer can comprise:

(i) an acrylamide monomer of the following Formula AM:

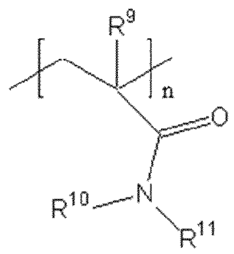

Formula AM

where $R^9$ is H or $C_{1-4}$ alkyl; and $R^{10}$ and $R^{11}$ are independently selected from the group consisting of H, $C_{1-4}$ alkyl, $CH_2OCH_3$, $CH_2OCH_2CH(CH_3)_2$, and phenyl, or together are $C_{3-6}$cycloalkyl; and

(ii) a cationic monomer conforming to Formula CM:

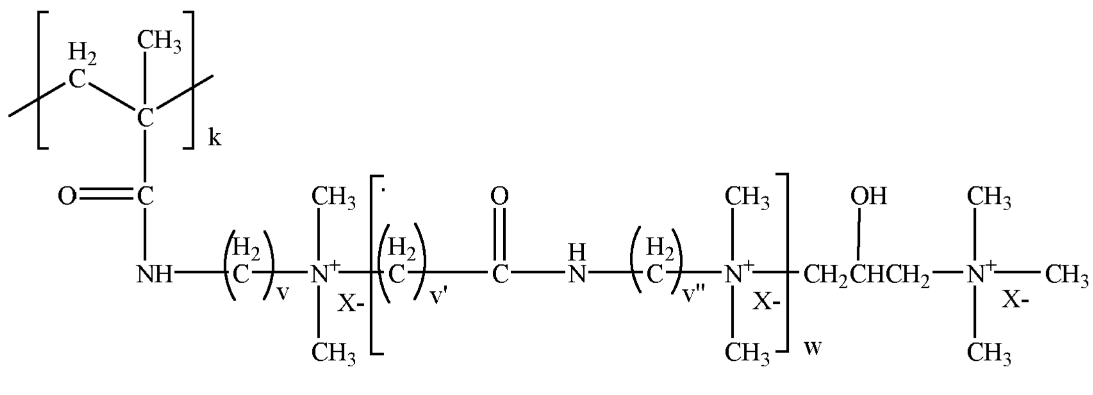

Formula CM

where k = 1, each of v, v', and v" is independently an integer of from 1 to 6, w is zero or an integer of from 1 to 10, and $X^-$ is an anion.

The cationic monomer can conform to Formula CM and where k = 1, v = 3 and w = 0, z = 1 and $X^-$ is $Cl^-$ to form the following structure:

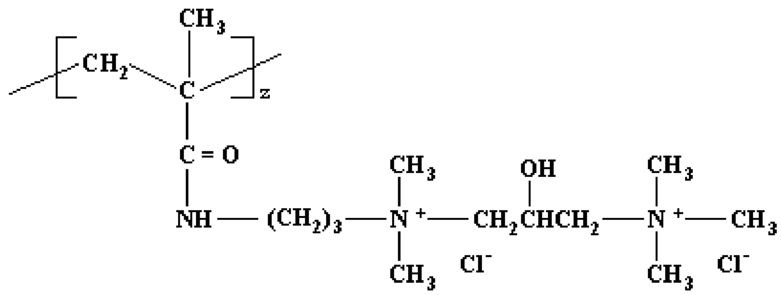

The above structure may be referred to as diquat. Alternatively, the cationic monomer can conform to Formula CM and wherein v and v" are each 3, v' = 1, w =1, y = 1 and $X^-$ is $Cl^-$, such as:

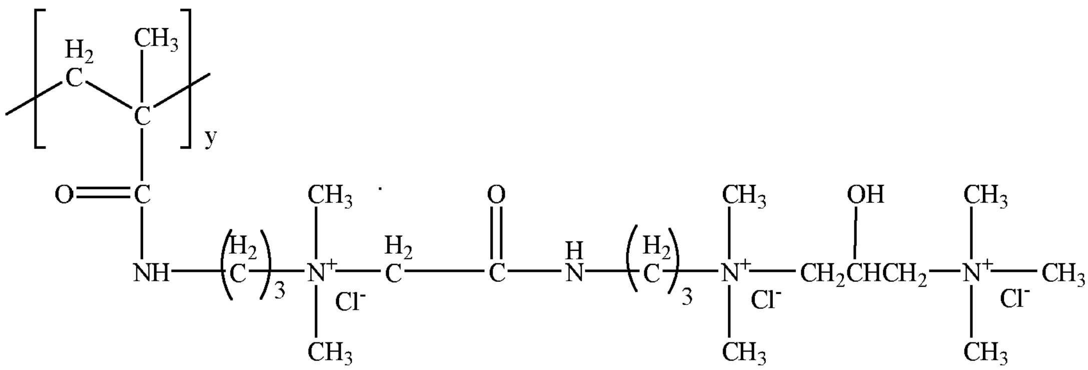

The above structure may be referred to as triquat.

Suitable acrylamide monomer include, but are not limited to, either acrylamide or methacrylamide.

The cationic copolymer (b) can be AM: TRIQUAT which is a copolymer of acrylamide and 1,3-Propanediaminium,N-[2-[[[dimethyl[3-[(2-methyl-1-oxo-2-propenyl)amino]propyl]ammonio]acetyl]amino]ethyl]2-hydroxy-N,N,N',N',N'-pentamethyl-, trichloride. AM:TRIQUAT is also known as polyquaternium 76 (PQ76). AM:TRIQUAT may have a charge density of 1.6 meq/g and a molecular weight of 1.1 million g/mol.

In an alternative embodiment, the cationic copolymer is of an acrylamide monomer and a cationic monomer, wherein the cationic monomer is selected from the group consisting of: dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide; ethylenimine, vinylamine, 2-vinylpyridine, 4- vinylpyridine; trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride, and mixtures thereof.

The cationic copolymer can comprise a cationic monomer selected from the group consisting of: cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, and mixtures thereof.

The cationic copolymer can be water-soluble. The cationic copolymer is formed from (1) copolymers of (meth)acrylamide and cationic monomers based on (meth)acrylamide, and/or hydrolysis-stable cationic monomers, (2) terpolymers of (meth)acrylamide, monomers based on cationic (meth)acrylic acid esters, and monomers based on (meth)acrylamide, and/or hydrolysis-stable cationic monomers. Monomers based on cationic (meth)acrylic acid esters may be cationized esters of the (meth)acrylic acid containing a quaternized N atom. In an embodiment, cationized esters of the (meth)acrylic acid containing a quaternized N atom are quaternized dialkylaminoalkyl (meth)acrylates with C1 to

C3 in the alkyl and alkylene groups. Suitable cationized esters of the (meth)acrylic acid containing a quaternized N atom can be selected from the group consisting of: ammonium salts of dimethylaminomethyl (meth)acrylate, dimethylaminoethyl (meth)acrylate, dimethylaminopropyl (meth)acrylate, diethylaminomethyl (meth)acrylate, diethylaminoethyl (meth)acrylate; and diethylaminopropyl (meth)acrylate quaternized with methyl chloride. In an embodiment, the cationized esters of the (meth)acrylic acid containing a quaternized N atom is dimethylaminoethyl acrylate, which is quaternized with an alkyl halide, or with methyl chloride or benzyl chloride or dimethyl sulfate (ADAME-Quat). the cationic monomer when based on (meth)acrylamides can be quaternized dialkylaminoalkyl(meth)acrylamides with C1 to C3 in the alkyl and alkylene groups, or dimethylaminopropylacrylamide, which is quaternized with an alkyl halide, or methyl chloride or benzyl chloride or dimethyl sulfate.

Suitable cationic monomer based on a (meth)acrylamide include quaternized dialkylaminoalkyl(meth)acrylamide with C1 to C3 in the alkyl and alkylene groups. The cationic monomer based on a (meth)acrylamide can be dimethylaminopropylacrylamide, which is quaternized with an alkyl halide, especially methyl chloride or benzyl chloride or dimethyl sulfate.

The cationic monomer can be a hydrolysis-stable cationic monomer. Hydrolysis-stable cationic monomers can be, in addition to a dialkylaminoalkyl(meth)acrylamide, all monomers that can be regarded as stable to the OECD hydrolysis test. The cationic monomer can be hydrolysis-stable and the hydrolysis-stable cationic monomer can be selected from the group consisting of: diallyldimethylammonium chloride and water-soluble, cationic styrene derivatives.

The cationic copolymer can be a terpolymer of acrylamide, 2-dimethylammoniumethyl (meth)acrylate quaternized with methyl chloride (ADAME-Q) and 3-dimethylammoniumpropyl(meth)acrylamide quaternized with methyl chloride (DIMAPA-Q). The cationic copolymer can be formed from acrylamide and acrylamidopropyltrimethylammonium chloride, wherein the acrylamidopropyltrimethylammonium chloride has a charge density of from 1.0 meq/g to 3.0 meq/g.

The cationic copolymer can have a charge density of from 1.1 meq/g to 2.5 meq/g, or from 1.1 meq/g to 2.3 meq/g, or from 1.2 meq/g to 2.2 meq/g, or from 1.2 meq/g to 2.1 meq/g, or from 1.3 meq/g to 2.0 meq/g, or from 1.3 meq/g to 1.9 meq/g.

The cationic copolymer can have a molecular weight from 100 thousand g/mol to 1.5 million g/mol, or from 300 thousand g/mol to 1.5 million g/mol, or from 500 thousand g/mol to 1.5 million g/mol, or from 700 thousand g/mol to 1.0 million g/mol, or from 900 thousand g/mol to 1.2 million g/mol.

The cationic copolymer can be a trimethylammoniopropylmethacrylamide chloride-N-Acrylamide copolymer, which is also known as AM:MAPTAC. AM:MAPTAC may have a charge density of 1.3 meq/g and a molecular weight of 1.1 million g/mol. The cationic copolymer can be AM:ATPAC. AM:ATPAC can have a charge density of 1.8 meq/g and a molecular weight of 1.1 million g/mol.

(a) Cationic Synthetic Polymers

The personal care composition can comprise a cationic synthetic polymer that may be formed from

i) one or more cationic monomer units, and optionally
ii) one or more monomer units bearing a negative charge, and/or
iii) a nonionic monomer,

wherein the subsequent charge of the copolymer is positive. The ratio of the three types of monomers is given by "m", "p" and "q" where "m" is the number of cationic monomers, "p" is the number of monomers bearing a negative charge and "q" is the number of nonionic monomers

The cationic polymers can be water soluble or dispersible, non-crosslinked, and synthetic cationic polymers having the following structure:

Monomer bearing a negative charge

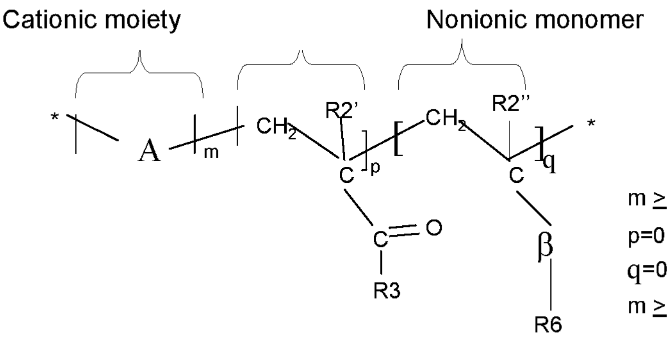


m ≥ 1
p=0 or ≥ 1
q=0 or ≥ 1
m ≥ p

where A, may be one or more of the following cationic moieties:

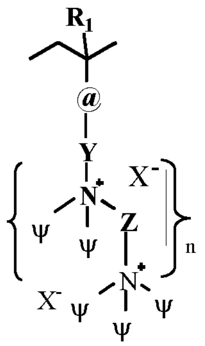
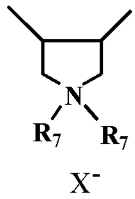
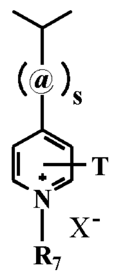

where @ = amido, alkylamido, ester, ether, alkyl or alkylaryl;
where Y = C1-C22 alkyl, alkoxy, alkylidene, alkyl or aryloxy;
where ψ = C1-C22 alkyl, alkyloxy, alkyl aryl or alkyl arylox;.
where Z = C1-C22 alkyl, alkyloxy, aryl or aryloxy;
where R1 = H, C1-C4 linear or branched alkyl;
where s = 0 or 1, n = 0 or ≥ 1;
where T and R7 = C1-C22 alkyl; and
where X- = halogen, hydroxide, alkoxide, sulfate or alkylsulfate.

Where the monomer bearing a negative charge is defined by R2' = H, C1-C4 linear or branched alkyl and R3 as:

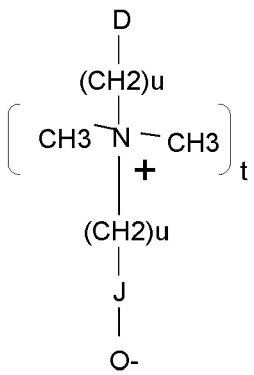
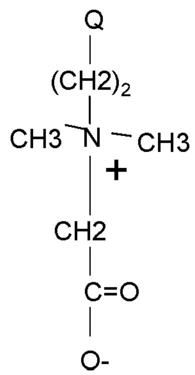
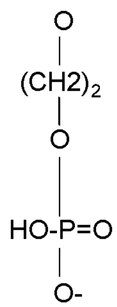
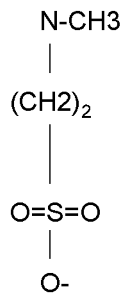

where D = O, N, or S;

where Q = $NH_2$ or O;

where u = 1-6;

where t = 0-1; and

where J = oxygenated functional group containing the following elements P, S, C.

Where the nonionic monomer is defined by R2" = H, C1-C4 linear or branched alkyl, R6 = linear or branched alkyl, alkyl aryl, aryl oxy, alkyloxy, alkylaryl oxy and β is defined as

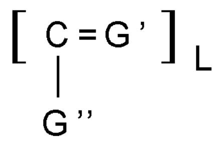

;

and where G' and G" are, independently of one another, O, S or N-H and L =0 or 1.

Examples of cationic monomers include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving from therefrom.

Further examples of cationic monomers include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.

Suitable cationic monomers include those which comprise a quaternary ammonium group of formula $-NR_3^+$, wherein R, which is identical or different, represents a hydrogen atom, an alkyl group comprising 1 to 10 carbon atoms, or a benzyl group, optionally carrying a hydroxyl group, and comprise an anion (counter-ion). Examples of anions are halides such as chlorides, bromides, sulphates, hydrosulphates, alkylsulphates (for example comprising 1 to 6 carbon atoms), phosphates, citrates, formates, and acetates.

Suitable cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride.

Additional suitable cationic monomers include trimethyl ammonium propyl (meth)acrylamido chloride.

Examples of monomers bearing a negative charge include alpha ethylenically unsaturated monomers comprising a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.

Suitable monomers with a negative charge include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Suitable nonionic monomers include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion (X- ) in association with the synthetic cationic polymers may be any known counterion so long as the polymers remain soluble or dispersible in water, in the hair care composition, or in a coacervate phase of the hair care composition, and so long as the counterions are physically and chemically compatible with the essential components of the hair care composition or do not otherwise unduly impair product performance, stability or aesthetics. Non limiting examples of such counterions include halides (e.g., chlorine, fluorine, bromine, iodine), sulfate and methylsulfate.

The cationic polymer described herein can aid in providing damaged hair, particularly chemically treated hair, with a surrogate hydrophobic F-layer. The microscopically thin F-layer provides natural weatherproofing, while helping to seal in moisture and prevent further damage. Chemical treatments damage the hair cuticle and strip away its protective F-layer. As the F-layer is stripped away, the hair becomes increasingly hydrophilic. It has been found that when lyotropic liquid crystals are applied to chemically treated hair, the hair becomes more hydrophobic and more virgin-like, in both look and feel. Without being limited to any theory, it is believed that the lyotropic liquid crystal complex creates a hydrophobic layer or film, which coats the hair fibers and protects the hair, much like the natural F-layer protects the hair. The hydrophobic layer returns the hair to a generally virgin-like, healthier state. Lyotropic liquid crystals are formed by combining the synthetic cationic polymers described herein with the aforementioned anionic detersive surfactant component of the hair care composition. The synthetic cationic polymer has a relatively high charge density. It should be noted that some synthetic polymers having a relatively high cationic charge density do not form lyotropic liquid crystals, primarily due to their abnormal linear charge densities. Such synthetic cationic polymers are described in WO 94/06403 to Reich et al. The synthetic polymers described herein can be formulated in a stable hair care composition that provides improved conditioning performance, with respect to damaged hair.

Cationic synthetic polymers that can form lyotropic liquid crystals have a cationic charge density of from 2 meq/gm to 7 meq/gm, and/or from 3 meq/gm to 7 meq/gm, and/or from 4 meq/gm to 7 meq/gm. In some embodiments, the cationic charge density is 6.2 meq/gm. The polymers also have a M. Wt. of from 1,000 to 5,000,000, and/or from 10,000 to 1,500,000, and/or from 100,000 to 1,500,000.

In another embodiment of the invention cationic synthetic polymers that provide enhanced conditioning and deposition of benefit agents but do not necessarily form lyotropic liquid crystals have a cationic charge density of from 0.7 meq/gm to 7 meq/gm, and/or from 0.8 meq/gm to 5 meq/gm, and/or from 1.0 meq/gm to 3 meq/gm. The polymers also have a M. Wt. of from 1,000 to 1,500,000, from 10,000 to 1,500,000, and from 100,000 to 1,500,000.

Suitable cationic cellulose polymers are salts of hydroxyethyl cellulose reacted with trimethyl ammonium substituted epoxide, referred to in the industry (CTFA) as Polyquaternium 10 and available from Dow/ Amerchol Corp. (Edison, N.J., USA) in their Polymer LR, JR, and KG series of polymers. Non-limiting examples include: JR-30M, KG-30M, JP, LR-400 and mixtures thereof. Other suitable types of cationic cellulose include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available from Dow/ Amerchol Corp. under the tradename SoftCAT Polymer SL-5, SoftCAT Polymer SL-30, Polymer SL-60, Polymer SL-100, Polymer SK-L, Polymer SK-M, Polymer SK-MH, and Polymer SK-H.

The concentration of the cationic polymers ranges 0.025% to 5%, from 0.1% to 3%, and/or from 0.2% to 1%, by weight of the personal care composition.

C. VISCOSITY REDUCING AGENTS

In an embodiment, the hair care composition described herein may comprise from 0.5% to 15%, alternatively from 0.75% to 10%, and alternatively from 1% to 7.5% of a viscosity reducing agent, by weight of the hair care composition. Non-limiting examples of suitable viscosity reducing agents include Class A materials, Class B materials, water miscible solvents, hydrotropes, silicone polyethers, and mixtures thereof.

The hair care composition described herein may have a liquid phase viscosity of from 8 centipoise to 15,000 centipoise, alternatively from 9 centipoise to 12,000 centipoise, alternatively from 10 centipoise to 11,000 centipoise, and alternatively from 11 centipoise to 8,000 centipoise, alternatively from 12 centipoise to 2,500 cps.

1. CLASS A MATERIALS

The Class A viscosity reducing agents may have a partition dispersion coefficient of from -5 to -0.7, alternatively from -4.6 to -0.85, alternatively from -4.5 to -0.9, alternatively from -3.1 to -0.7, and alternatively from -3 to -0.85. The Class A viscosity reducing agents may have a partition dispersion coefficient of from -4.6 to -1.9, alternatively from -4.5 to -2, wherein the one or more viscosity reducing agents has at least 2 polar groups, or has 1 polar group and less than 5 acyclic sp3 hybridized carbon atoms that are connected to each other in a contiguous group. The Class A viscosity reducing agents may have a partition dispersion coefficient of from -4.6 to -1.9, alternatively from - 4.5 to -2, wherein the one or more viscosity reducing agents has 2 to 4 polar groups, or has 1 polar group and 1 to 3 acyclic sp3 hybridized carbon atoms that are connected to each other in a contiguous group. The Class A viscosity reducing agents may have a partition dispersion coefficient of from -4.6 to -1.9, alternatively from -4.5 to -2, wherein the one or more viscosity reducing agents has 2 to 4 polar groups, or has 1 polar group and 2 acyclic sp3 hybridized carbon atoms that are

connected to each other in a contiguous group. The Class A viscosity reducing agents may provide unexpected viscosity reduction when used in the hair care composition described herein.

The partition dispersion coefficient (PDC) is defined by the following equation:

$$PDC= \log P - 0.3001 * (\delta D)2 + 10.362 * \delta D - 93.251$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein δD is the Hansen solubility dispersion parameter in (MPa)1/2 computed using Steven Abbott and Hiroshi Yamamoto's "HSPIP - Hansen Solubility Parameters in Practice" program, 4th Edition, version 4.1.07.

The viscosity reducing agents may be organic compounds comprising 1 polar group, alternatively at least 1 polar group, alternatively 2 to 4 polar groups, and alternatively at least 2 polar groups. The polar groups may be selected from the group consisting of alcohols, aldehydes, esters, lactones, coumarins, ethers, ketones, phenol, phenyl, oxides, alkenyl, alkynyl, and combinations thereof. The viscosity reducing agents may have a molecular weight of between 100 daltons and 300 daltons, alternatively from 125 daltons to 300 daltons. Additionally, the viscosity reducing agents may have a water solubility at between 23 and 25 degrees Celsius of from 900 to 50,000 mg/L.

The Class A viscosity reducing agents may be selected from the group consisting of raspberry ketone, triethyl citrate, 5-methyl-3-heptanone oxime, hydroxycitronellal, camphor gum, 2-isopropyl-5-methyl-2-hexenal, eucalyptol, 1,1-dimethoxyoctane, isobutyl hexanoate, dihydro iso jasmonate, and combinations thereof. Alternatively, the viscosity reducing agents may be selected from the group consisting of raspberry ketone, triethyl citrate, hydroxycitronellal, ethanol, dipropylene glycol, and combinations thereof.

2. CLASS B MATERIALS

The Class B viscosity reducing agents may have a partition dispersion coefficient of from 0.05 to 5.1, alternatively from 0.08 to 4.5, alternatively from 0.09 to 4.4, alternatively from 0.05 to 2.0, alternatively from 0.08 to 1.8, alternatively from 0.09 to 1.7, and alternatively from 0.095 to 1.68. The Class B viscosity reducing agents may provide unexpected viscosity reduction when used in the hair care composition described herein.

The partition dispersion coefficient (PDC) is defined by the following equation:

$$PDC= \log P - 0.3001 * (\delta D)^2 + 10.362 * \delta D - 93.251$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein δD is the Hansen solubility dispersion parameter in $(MPa)^{1/2}$ computed using Steven Abbott and Hiroshi Yamamoto's "HSPIP - Hansen Solubility Parameters in Practice" program, 4th Edition, version 4.1.07.

The viscosity reducing agents may be organic compounds comprising 1 polar group, alternatively at least 1 polar group, alternatively 2 to 4 polar groups, and alternatively at least 2 polar groups. The polar groups may be selected from the group consisting of alcohols, aldehydes, esters, lactones, coumarins, ethers, ketones, phenol, phenyl, oxides, alkenyl, alkynyl, and combinations thereof. The viscosity reducing agents may have a molecular weight of between 100 daltons and 300 daltons, alternatively from 125 daltons to 300 daltons. Additionally, the viscosity reducing agents may have a water solubility at between 23 and 25 degrees Celsius of from 10 to 900 mg/L.

The Class B viscosity reducing agents may be selected from the group consisting of veloutone, isoamyl salicylate, gamma-terpinene, linalyl iso butyrate, alpha-terpinene, limonene, dipentene, geranyl phenyl acetate, iso propyl myristate, hexadecane, and combinations thereof. Alternatively, the Class B viscosity reducing agents may be selected from the group consisting of veloutone, gamma-terpinene, linalyl iso butyrate, alpha-terpinene, limonene, dipentene, geranyl phenyl acetate, iso propyl myristate, hexadecane, and combinations thereof. Alternatively, the Class B viscosity reducing agents may be selected from the group consisting of veloutone, isoamyl salicylate, gamma-terpinene, linalyl iso butyrate, alpha-terpinene, limonene, dipentene, geranyl phenyl acetate, and combinations thereof.

3. WATER MISCIBLE SOLVENTS

The carrier useful in embodiments of the personal care composition includes water and water solutions of lower alkyl alcohols, polyhydric alcohols, ketones having from 3 to 4 carbons atoms, C1-C6 esters of C1-C6 alcohols, sulfoxides, amides, carbonate esters, ethoxylated and proposylated C1-C10 alcohols, lactones, pyrollidones, and mistures thereof. Non-limited lower alkyl alcohol examples are monohydric alcohols having 1 to 6 carbons, such as ethanol and isopropanol.

Non-limiting examples of polyhydric alcohols useful herein include propylene glycol, dipropylene glycol, butylenes glycol, hexylene glycol, glycerin, propane diol and mixtures thereof.

In an embodiment of the present invention, the personal care composition may comprise a hydrotrope/viscosity modifier which is an alkali metal or ammonium salt of a lower alkyl benzene sulphonate such as sodium xylene sulphonate, sodium cumene sulphonate or sodium toluene sulphonate.

In a further embodiment of the present invention, the personal care composition may comprise silicone polyethers. Non-limiting examples include PEG-8 Dimethicones with molecular weights between 500 g/mol and 3500g/mol including, Silsurf A208 (molecular weight of 855 g/mol) and Silsurf D208 (molecular weight of 2706 g/mol).

D. PROPELLANT OR BLOWING AGENT

The concentrated personal care composition described herein may comprise from from 1% to 10% propellant or blowing agent, alternatively from 2% to 8% propellant or blowing agent, by weight of the concentrated personal care composition.

The propellant or blowing agent may comprise one or more volatile materials, which in a gaseous state, may carry the other components of the concentrated personal care composition in particulate or droplet form or as a foam. The propellant or blowing agent may have a boiling point within the range of from -45° C. to 5° C. The propellant or blowing agent may be liquefied when packaged in convention aerosol containers under pressure. The rapid boiling of the propellant or blowing agent upon leaving the aerosol foam dispenser may aid in the atomization or foaming of the other components of the concentrated personal care composition.

Aerosol propellants or blowing agents which may be employed in the aerosol composition may include the chemically-inert hydrocarbons such as propane, n-butane, isobutane, cyclopropane, and mixtures thereof, as well as halogenated hydrocarbons such as dichlorodifluoromethane, 1,1-dichloro-1,1,2,2-tetrafluoroethane, 1 -chloro-1,1 -difluoro-2,2-trifluoroethane, 1-chloro-1,1-difluoroethylene, 1,1-difluoroethane, dimethyl ether, monochlorodifluoromethane, trans-1,3,3,3-tetrafluoropropene, and mixtures thereof. The propellant or blowing agent may comprise hydrocarbons such as isobutane, propane, and butane-these materials may be used for their low ozone reactivity and may be used as individual components where their vapor pressures at 21.1° C. range from 1.17 Bar to 7.45 Bar, alternatively from 1.17 Bar to 4.83 Bar, and alternatively from 2.14 Bar to 3.79 Bar.

E. SCALP HEALTH AGENTS

In an embodiment of the present invention, one or more scalp health agent may be added to provide scalp benefits in addition to the anti-fungal/anti-dandruff efficacy provided by the surfactant soluble anti-dandruff agents. This group of materials is varied and provides a wide range of benefits including moisturization, barrier improvement, anti-fungal, anti-microbial and anti-oxidant, anti-itch, and sensates, and additional anti-dandruff agents such as zinc pyrithione (ZPT) or selenium sulfide. Such scalp health agents include but are not limited to: vitamin E and F, salicylic acid, niacinamide, caffeine, panthenol, zinc oxide, zinc carbonate, glycols, glycolic acid, PCA, PEGs, erythritol, glycerin, triclosan, lactates, hyaluronates, allantoin and other ureas, betaines, sorbitol, glutamates, xylitols, menthol, menthyl lactate, iso cyclomone, benzyl alcohol, a compound comprising the following structure:

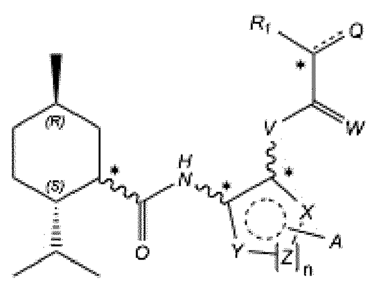

$R_1$ is selected from H, alkyl, amino alkyl, alkoxy;
Q = $H_2$, O, $-OR_1$, $-N(R_1)_2$, $-OPO(OR_1)_x$, $-PO(OR_1)_x$, $-P(OR_1)_x$ where x = 1-2;
V = $NR_1$, O, $-OPO(OR_1)_x$, $-PO(OR_1)_x$, $-P(OR_1)_x$ where x = 1-2;
W = $H_2$, O;
X, Y = independently selected from H, aryl, naphthyl for n=0;
X, Y = aliphatic $CH_2$ or aromatic CH for n ≥ 1 and Z is selected from aliphatic $CH_2$, aromatic CH, or heteroatom;
A = lower alkoxy, lower alkylthio, aryl, subsitituted aryl or fused aryl; and
stereochemistry is variable at the positions marked*.

and natural extracts/oils including peppermint, spearmint, argan, jojoba and aloe.

F. OPTIONAL INGREDIENTS

In accordance with embodiments of the present invention, the personal care composition may further comprise one or more optional ingredients, including benefit agents Suitable benefit agents include, but are not limited to conditioning agents, cationic polymers silicone emulsions, anti-dandruff agents, gel networks, chelating agents, and, natural oils such as sun flower oil or castor oil. Additional suitable optional ingredients include but are not limited to perfumes, perfume microcapsules, colorants, particles, anti-microbials, foam busters, anti-static agents, rheology modifiers and thickeners, suspension materials and structurants, pH adjusting agents and buffers, preservatives, pearlescent agents, solvents, diluents, anti-oxidants, vitamins and combinations thereof.

Such optional ingredients should be physically and chemically compatible with the components of the composition, and should not otherwise unduly impair product stability, aesthetics, or performance. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein.

1. Conditioning Agents

The conditioning agent of the personal care compositions can be a silicone conditioning agent. The silicone conditioning agent may comprise volatile silicone, non-volatile silicone, or combinations thereof. The concentration of the silicone conditioning agent typically ranges from 0.01% to 10%, by weight of the composition, from 0.1% to 8%, from 0.1% to 5%, and/or from 0.2% to 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609.

The silicone conditioning agents for use in the compositions of the present invention can have a viscosity, as measured at 25° C, from 0.00002 to 2 $m^2/s$ (from 20 to 2,000,000 centistokes ("csk")), from 0.001 to 1.8 $m^2/s$ (from 1,000 to 1,800,000 csk), from 0.01 to 1.5 $m^2/s$ (from 10,000 to 1,500,000 csk), and/or from 0.02 to 1.5 $m^2/s$ (from 20,000 to 1,500,000 csk).

The dispersed silicone conditioning agent particles typically have a volume average particle diameter ranging from 0.01 micrometer to 60 micrometer. For small particle application to hair, the volume average particle diameters typically range from 0.01 micrometer to 4 micrometer, from 0.01 micrometer to 2 micrometer, from 0.01 micrometer to 0.5 micrometer.

Additional material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

Silicone emulsions suitable for use in the embodiments of the present invention include, but are not limited to, emulsions of insoluble polysiloxanes prepared in accordance with the descriptions provided in U.S. Patent No. 6,316,541 or U.S. Patent No. 4,476,282 or U.S. Patent Application Publication No. 2007/0276087. Accordingly, suitable insoluble polysiloxanes include polysiloxanes such as alpha, omega hydroxy-terminated polysiloxanes or alpha, omega alkoxy-terminated polysiloxanes having an internal phase viscosity from 0.000005 $m^2/s$ to 0.5 $m^2/s$ (from 5 csk to 500,000 csk). For example, the insoluble polysiloxane may have an internal phase viscosity less than 0.4 $m^2/s$ (less than 400,000 csk), preferably less than 0.2 $m^2/s$ (less than 200,000 csk), more preferably from 0.01 $m^2/s$ to 0.18 $m^2/s$ (from 10,000 csk to 180,000 csk). The insoluble polysiloxane can have an average particle size within the range from 10 nm to 10 micron. The average particle size may be within the range from 15 nm to 5 micron, from 20nm to 1 micron, or from 25 nm to 500 nm.

The average molecular weight of the insoluble polysiloxane, the internal phase viscosity of the insoluble polysiloxane, the viscosity of the silicone emulsion, and the size of the particle comprising the insoluble polysiloxane are determined by methods commonly used by those skilled in the art, such as the methods disclosed in Smith, A. L. The Analytical Chemistry of Silicones, John Wiley & Sons, Inc.: New York, 1991. For example, the viscosity of the silicone emulsion can be measured at 30°C with a Brookfield viscometer with spindle 6 at 2.5 rpm. The silicone emulsion may further include an additional emulsifier together with the anionic surfactant,

Other classes of silicones suitable for use in compositions of the present invention include but are not limited to: i) silicone fluids, including but not limited to, silicone oils, which are flowable materials having viscosity less than 1 $m^2/s$ (less than 1,000,000 csk) rst as measured at 25°C; ii) aminosilicones, which contain at least one primary, secondary or tertiary amine; iii) cationic silicones, which contain at least one quaternary ammonium functional group; iv) silicone gums; which include materials having viscosity greater than or equal to 1 $m^2/s$ (greater than or equal to 1,000,000 csk csk) as measured at 25°C; v) silicone resins, which include highly cross-linked polymeric siloxane systems; vi) high refractive index silicones, having refractive index of at least 1.46, and vii) mixtures thereof.

The conditioning agent of the personal care compositions of the present invention may also comprise at least one organic conditioning material such as oil or wax, either alone or in combination with other conditioning agents, such as the silicones described above. The organic material can be non-polymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to 2,000,000 including those with CTFA names PEG-200, PEG-400, PEG-600, PEG-1000, PEG-2M, PEG-7M, PEG-14M, PEG-45M and mixtures thereof.

2. Emusifiers

A variety of anionic and nonionic emulsifiers can be used in the personal care composition of the present invention. The anionic and nonionic emulsifiers can be either monomeric or polymeric in nature. Monomeric examples include, by way of illustrating and not limitation, alkyl ethoxylates, alkyl sulfates, soaps, and fatty esters and their derivatives. Polymeric examples include, by way of illustrating and not limitation, polyacrylates , polyethylene glycols, and block copolymers and their derivatives. Naturally occurring emulsifiers such as lanolins, lecithin and lignin and their derivatives are also non-limiting examples of useful emulsifiers.

3. Chelating Agents

The personal care composition can also comprise a chelant. Suitable chelants include those listed in A E Martell & R M Smith, Critical Stability Constants, Vol. 1, Plenum Press, New York & London (1974) and A E Martell & R D Hancock, Metal Complexes in Aqueous Solution, Plenum Press, New York & London (1996). When related to chelants, the term "salts and derivatives thereof" means the salts and derivatives comprising the same functional structure (e.g., same chemical backbone) as the chelant they are referring to and that have similar or better chelating properties. This term include alkali metal, alkaline earth, ammonium, substituted ammonium (i.e. monoethanolammonium, diethanolammonium, triethanolammonium) salts, esters of chelants having an acidic moiety and mixtures thereof, in particular all sodium, potassium or ammonium salts. The term "derivatives" also includes "chelating surfactant" compounds, such as those exemplified in U.S. Pat. No. 5,284,972, and large molecules comprising one or more chelating groups having the same functional structure as the parent chelants, such as polymeric EDDS (ethylenediaminedisuccinic acid) disclosed in U.S. Pat. No. 5,747,440.

Levels of the EDDS chelant in the personal care compositions can be as low as 0.01 wt% or even as high as 10 wt%, but above the higher level (i.e., 10 wt%) formulation and/or human safety concerns may arise. In an embodiment, the level of the EDDS chelant may be at least 0.05 wt%, at least 0.1 wt%, at least 0.25 wt%, at least 0.5 wt%, at least 1 wt%, or at least 2 wt% by weight of the personal care composition. Levels above 4 wt% can be used but may not result in additional benefit.

4. Aqueous Carrier

The personal care compositions can be in the form of pourable liquids (under ambient conditions). Such compositions will therefore typically comprise a carrier, which is present at a level of from 40% to 85%, alternatively from 45% to 80%, alternatively from 50% to 75% by weight of the personal care composition. The carrier may comprise water, or a miscible mixture of water and organic solvent, and in one aspect may comprise water with minimal or no significant concentrations of organic solvent, except as otherwise incidentally incorporated into the composition as minor ingredients of other essential or optional components.

The carrier useful in embodiments of the personal care compositions of the present invention includes water and water solutions of lower alkyl alcohols and polyhydric alcohols. The lower alkyl alcohols useful herein are monohydric alcohols having 1 to 6 carbons, in one aspect, ethanol and isopropanol. Exemplary polyhydric alcohols useful herein include propylene glycol, hexylene glycol, glycerin, and propane diol.

G. FOAM DISPENSER

The personal care composition described herein may be provided in a foam dispenser. The foam dispenser may be an aerosol foam dispenser. The aerosol foam dispenser may comprise a reservoir for holding the personal care treatment composition. The reservoir may be made out of any suitable material selected from the group consisting of plastic, metal, alloy, laminate, and combinations thereof. In an embodiment, the reservoir may be for one-time use. In an embodiment, the reservoir may be removable from the aerosol foam dispenser. Alternatively, the reservoir may be

integrated with the aerosol foam dispenser. In an embodiment, there may be two or more reservoirs.

The foam dispenser may also be a mechanical foam dispenser. The mechanical foam dispenser described may be selected from the group consisting of squeeze foam dispensers, pump foam dispensers, other mechanical foam dispensers, and combinations thereof. In an embodiment, the mechanical foam dispenser is a squeeze foam dispenser. Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706).

The mechanical foam dispenser may comprise a reservoir for holding the concentrated personal treatment composition. The reservoir may be made out of any suitable material selected from the group consisting of plastic, metal, alloy, laminate, and combinations thereof. The reservoir may be a refillable reservoir such as a pour-in or screw-on reservoir, or the reservoir may be for one-time use. The reservoir may also be removable from the mechanical foam dispenser. Alternatively, the reservoir may be integrated with the mechanical foam dispenser. In an embodiment, there may be two or more reservoirs.

In an embodiment, the reservoir may be comprised of a material selected from the group consisting of rigid materials, flexible materials, and combinations thereof. The reservoir may be comprised of a rigid material if it does not collapse under external atmospheric pressure when it is subject to an interior partial vacuum.

H. PRODUCT FORM

The personal care compositions of the present invention may be presented in typical hair care formulations. They may be in the form of solutions, dispersion, emulsions, powders, talcs, encapsulated, spheres, spongers, solid dosage forms, foams, and other delivery mechanisms. The compositions of the embodiments of the present invention may be hair tonics, leave-on hair products such as treatment, and styling products, rinse-off hair products such as shampoos and personal cleansing products, and treatment products; and any other form that may be applied to hair.

I. APPLICATOR

In an embodiment of the present invention, the personal care composition may be dispensed from an applicator for dispensing directly to the scalp area. Dispensing directly onto the scalp via a targeted delivery applicator enables deposition of the non diluted cleaning agents directly where the cleaning needs are highest. This also minimizes the risk of eye contact with the cleansing solution.

The applicator is attached or can be attached to a bottle containing the cleansing composition. The applicator can consist of a base that holds or extends to a single or plurality of tines. The tines have openings that may be at the tip, the base or at any point between the tip and the base. These openings allows for the product to be distributed from the bottle directly onto the hair and/or scalp.

Alternatively, the applicator can also consist of brush-like bristles attached or extending from a base. In this case product would dispense from the base and the bristles would allow for product distribution via the combing or brushing motion.

Applicator and tine design and materials can also be optimized to enable scalp massage. In this case it would be beneficial for the tine or bristle geometry at the tips to be more rounded similar to the roller ball applicator used for eye creams. It may also be beneficial for materials to be smoother and softer; for example metal or metal-like finishes, "rubbery materials".

**Preparation of Shampoo Compositions**

In an embodiment of the present invention, shampoo compositions are prepared by adding surfactants, surfactant soluble agents, perfume, viscosity modifiers, cationic polymers and the remainder of the water with ample agitation to ensure a homogenous mixture. The mixture can be heated to 50-75°C to speed the solubilization of the soluble agents, then cooled. Product pH may be adjusted as necessary to provide shampoo compositions of the present invention which are suitable for application to human hair and scalp, and may vary based on the selection of particular detersive surfactants and/or other components.

**Viscosity Measurement**

Shampoo viscosities can be measured on a 2.5 mL sample using a cone and plate Brookfield RS rheometer with cone C75-1 at 2 $s^{-1}$, 27°C at 3 mins.

**Measurement of Diffusion Coefficients by NMR**

Surfactant-soluble agent containing cleansing compositions are diluted with deuterium oxide to a surfactant concentration of 1.3%. This dilution factor is believed to be representative of a cleansing composition when applied to a head during use. The diluted samples are introduced into 5mm NMR tubes with no further preparation. All experiments are run locked on $D_2O$ at 25°C. Diffusion coefficients are determined using the vendor-supplied pulse sequence ledbpgp2s (stimulated echo with bipolar gradients, longitudinal eddy current delay and 2 spoil gradients) using a Bruker Avance 700 MHz NMR spectrometer equipped with a BBO z gradient probe. Gradient pulse durations ranged between 5000 - 6000 μs, with diffusion periods set at 150 ms. Thirty two exponentially-spaced gradient values are used ranging from 2%-95% of 10 A current from a GREAT 3/10 amplifier, with resulting gradient strengths given by 5 Gauss/cm/A. Data are processed using vendor supplied software fitting to a single component.

The surfactant micelle diffusion coefficient is designated as $D_S$ and the surfactant-soluble agent diffusion coefficient is designated as $D_A$. The ratio of the surfactant diffusion coefficient to the surfactant-soluble agent diffusion coefficient can be calculated using the following equation:

$$Ratio\ of\ Diffusion\ Coefficients = \frac{D_S}{D_A}$$

**Measurement of Surfactant-Soluble Agent Deposition**

Surfactant soluble agent deposition in-vivo on scalp can be determined by ethanol extraction of the agent after the scalp has been treated with a surfactant-soluble agent containing cleansing composition, for example apply 10 grams of cleansing composition per person's head, lather for 1 minute, and then rinse off. The concentration of agent in the ethanol extraction solvent is measured by HPLC. Quantitation is made by reference to a standard curve. The concentration detected by HPLC is converted into an amount collected in grams by using the concentration multiplied by volume of extraction solvent.

The percent agent deposited on scalp can be calculated using the following equation: *% agent deposited on scalp*

$$= \frac{\frac{grams\ of\ agent\ deposited}{area\ of\ scalp\ extracted}}{\frac{(wt.\%\ agent\ in\ composition) \times (grams\ of\ composition\ applied)}{area\ of\ scalp\ treated}} \times 100\%$$

Deposition of volatile surfactant soluble agents such as perfume on hair can be determined by headspace analysis using solid-phase micro-extraction (SPME) gas chromatography/mass spectrometry (GC-MS). After hair has been treated with a surfactant-soluble agent containing cleansing composition, for example wet hair, apply 0.1 gram of cleansing composition per gram of hair, lather for 30 seconds and then rinse off for 30 seconds, the hair is placed into a clean 125 mL I-Chem jar, closed with PTFE septum caps, and allowed to equilibrate for at least 30 minutes at 40C. The samples (neat or solution) are then analyzed using the automated SPME-GC-MS analysis system. Perfume compounds can be identified using the MS spectral libraries of John Wiley & Sons and the National Institute of Standards and Technology (NIST), purchased and licensed through Agilent. Chromatographic peaks for specific ions are integrated using the MassHunter software obtained from Agilent Technologies, Inc., Wilmington, DE, USA and reported as an average area. The average area reported correlates to the amount of surfactant soluble agent deposited on hair.

The average area per gram of agent applied can be calculated using the following equation: *average area per gram of agent*

$$= \frac{average\ area\ reported}{(wt.\%\ agent\ in\ composition) \times (grams\ of\ composition\ applied\ on\ hair)}$$

The deposition efficiency can be calculated using one of the following equations:

$$Deposition\ efficiency = \frac{\%\ agent\ deposited\ by\ example\ formula}{\%\ agent\ deposited\ by\ control\ formula}$$

$$Deposition\ efficiency = \frac{average\ area\ per\ gram\ of\ agent\ from\ example\ formula}{average\ area\ per\ gram\ of\ agent\ from\ control\ formula}$$

**Non-limiting Examples**

The shampoo compositions illustrated in the following examples are prepared by conventional formulation and mixing methods. All exemplified amounts are listed as weight percents on an active basis and exclude minor materials such as diluents, preservatives, color solutions, imagery ingredients, botanicals, and so forth, unless otherwise specified. All percentages are based on weight unless otherwise specified.

Examples 1-24 are not within the scope of the recited claims.

| Ingredient | Examples, active wt% | |
|---|---|---|
| | 1 (control) | 2 |
| Water | q. s. | q. s. |
| Sodium Laureth-1 Sulfate (SLE1S)[1] | 14.00 | - |
| Sodium Undecyl Sulfate [2] | - | 14.00 |
| Piroctone Olamine [3] (ClogP = 3.5) | 1.00 | 1.00 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 1.15E-10 | 1.78E-10 |
| Diffusion Coefficient of Surfactant-soluble agent, $D_A$ ($m^2/s$) | 1.07E-10 | 1.18E-10 |
| Ratio of Diffusion Coefficients, $D_S/D_A$ | 1.1 | 1.5 |
| | | |
| % Piroctone Olamine deposited | 1.1% | 1.9% |
| Deposition Efficiency (vs control) | 1.0X | 1.7X |

1 Sodium Laureth-1 Sulfate at 26% active, supplier: P&G
2 Sodium Undecyl Sulfate at 70% active, supplier: P&G
3 Octopirox, supplier: Clariant
4 Sodium Chloride, supplier: Morton

**Discussion of Results for Examples 1-2**

For Example 1 (control), the ratio of diffusion coefficients ($D_S/D_A$) is close to 1.0 which indicates that the Piroctone Olamine is diffusing at the same rate as the SLE1S micelles, which allows one to infer that the Piroctone Olamine is within the SLE1S micelle. However, in Example 2 where the SLE1S is replaced with Sodium Undecyl Sulfate, the ratio ($D_S/D_A$) is greater than 1.0. This change in $D_S/D_A$ indicates that the Piroctone Olamine is diffusing at a different rate than the Sodium Undecyl Sulfate micelles, which allows one to infer that the Piroctone Olamine is not within those micelles. The implications of the Piroctone Olamine not being within the surfactant micelles in Example 1 is reflected in the increased deposition efficiency of Example 2 vs. Example 1 (control).

| Ingredient | Examples, active wt% | | |
|---|---|---|---|
| | 3 (control) | 4 | 5 |
| Water | q. s. | q. s. | q. s. |
| Sodium Laureth-1 Sulfate (SLE1S)[1] | 14.00 | - | - |
| Sodium Undecyl Sulfate [2] | - | 14.00 | 28.00 |

(continued)

| Ingredient | Examples, active wt% | | |
|---|---|---|---|
| | 3 (control) | 4 | 5 |
| Climbazole [3] (ClogP = 3.8) | 1.00 | 1.00 | 2.00 |
| Sodium Chloride [4] | Up to 2% | Up to 2% | Up to 2% |
| Preservatives, pH adjusters | Up to 1% | Up to 1% | Up to 1% |
| | | | |
| Diffusion Coefficient of Surfactant, $D_S$ ($m^2/s$) | 8.50E-11 | 2.18E-10 | 2.05E-10 |
| Diffusion Coefficient of Surfactant-soluble agent, $D_A$ ($m^2/s$) | 7.26E-11 | 9.83E-11 | 1.02E-10 |
| Ratio of Diffusion Coefficients, $D_S/D_A$ | 1.2 | 2.2 | 2.0 |
| | | | |
| % Climbazole deposited | 0.57% | 1.1% | 1.3% |
| Deposition Efficiency (vs control) | 1.0X | 1.9X | 2.5X |
| 1 Sodium Laureth-1 Sulfate at 26% active, supplier: P&G<br>2 Sodium Undecyl Sulfate at 70% active, supplier: P&G<br>3 Climbazole, supplier: Symrise<br>4 Sodium Chloride, supplier: Morton | | | |

**Discussion of Results for Examples 3-5**

For Example 3 (control), the ratio of diffusion coefficients ($D_S/D_A$) is close to 1.0 which indicates that the Climbazole is diffusing at the same rate as the SLE1S micelles, which allows one to infer that the Climbazole is within the SLE1S micelle. However, in Example 4-5 where the SLE1S is replaced with Sodium Undecyl Sulfate, the ratio ($D_S/D_A$) is significantly greater than 1.0. This change in $D_S/D_A$ indicates that the Climbazole is diffusing at a different rate than the Sodium Undecyl Sulfate micelles, which allows one to infer that the Climbazole is not within those micelles. Consequently, Examples 4-5 are representative of the present invention and exhibit significantly greater deposition efficiency which is 1.9X-2.5X that of Example 3 (control). This demonstrates that the present invention can be broadly applied to a variety of soluble anti-dandruff agents.

| Ingredient | Examples, active wt% | | | | |
|---|---|---|---|---|---|
| | 6 (control) | 7 | 8 | 9 | 10 (com parati ve) |
| Water | q. s. | q. s. | q. s. | q. s. | q. s. |
| Sodium Laureth-1 Sulfate (SLE1S)[1] | 14.00 | 16.00 | 16.00 | - | 21.60 |
| Sodium Trideceth-2 Sulfate [2] | - | - | - | 16.00 | - |
| Sodium Undecyl Sulfate [3] | - | 8.00 | - | - | 2.40 |
| Sodium Decyl Sulfate [4] | - | - | 8.00 | 8.00 | - |
| Piroctone Olamine [5] (ClogP = 3.5) | 1.00 | 2.00 | 2.00 | 2.00 | 2.00 |
| Sodium Chloride [6] | Up to 2% | Up to 2% | Up to 2% | Up to 2% | Up to 2% |
| Preservatives, fragrance, pH adjusters | Up to 2.5% | Up to 2.5% | Up to 2.5% | Up to 2.5% | Up to 2.5% |
| | | | | | |
| Diffusion Coefficient of Surfactant, $D_S$ ($m^2/s$) | 1.10E-10 | 1.21E-10 | 1.55E-10 | 2.89E-10 | 1.21 E-10 |
| Diffusion Coefficient of Surfactant-soluble agent, $D_A$ ($m^2/s$) | 9.05E-11 | 8.57E-11 | 1.07E-10 | 1.36E-10 | 1.03 E-10 |
| Ratio of Diffusion Coefficients, $D_S/D_A$ | 1.2 | 1.4 | 1.4 | 2.1 | 1.2 |

(continued)

| Ingredient | Examples, active wt% | | | | |
|---|---|---|---|---|---|
| | 6 (control) | 7 | 8 | 9 | 10 (com parati ve) |
| | | | | | |
| % Piroctone Olamine deposited | 2.1% | 3.1% | 3.0% | 4.2% | 2.4% |
| Deposition Efficiency (vs control) | 1.0X | 1.5X | 1.4X | 2.0X | 1.1X |

1 Sodium Laureth-1 Sulfate at 26% active, supplier: P&G
2 Sodium Trideceth-2 Sulfate at 65% active, supplier: Tianjin Tianzhi Fine Chemical Co
3 Sodium Undecyl Sulfate at 70% active, supplier P&G
4 Sodium Decyl Sulfate at 70% active, supplier: P&G
5 Octopirox, supplier: Clariant
6 Sodium Chloride, supplier: Morton

**Discussion of Results for Examples 6-10**

This set of examples demonstrates that achieving diffusion coefficient ratios ($D_S/D_A$) greater than 1.2 is a key component to achieving the desired increased deposition efficiencies. Examples 7-9 are representative of the present invention and demonstrate that it is possible to achieve diffusion coefficient ratios ($D_S/D_A$) greater than 1.2 with a variety of surfactants. Consequently, Examples 7-9 exhibit greater deposition efficiencies (1.4-2.0X) than that of Example 6 (control). Example 10 is a comparative example and shows that even when a formula contains Sodium Undecyl Sulfate, if $D_S/D_A$ is close 1.0 the deposition efficiency will be low and similar to that of the control (Example 6).

| Ingredient | Examples, active wt% | |
|---|---|---|
| | 11 (control) | 12 |
| Water | q. s. | q. s. |
| Sodium Laureth-1 Sulfate (SLE1S)[1] | 14.00 | - |
| Sodium Decyl Sulfate [2] | - | 14.00 |
| Hexyl Cinnamic Aldehyde [3] (ClogP = 4.3) | 0.30 | 0.30 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 5.53E-11 | 2.69E-10 |
| Diffusion Coefficient of Surfactant-soluble agent, $D_A$ ($m^2/s$) | 6.31E-11 | 8.60E-11 |
| Ratio of Diffusion Coefficients, $D_S/D_A$ | 0.88 | 3.1 |
| | | |
| Average area per gram of Hexyl Cinnamic Aldehyde | 2.35E+04 | 1.10E+05 |
| Deposition Efficiency (vs control) | 1.0X | 4.7X |

1 Sodium Laureth-1 Sulfate at 26% active, supplier: P&G
2 Sodium Undecyl Sulfate at 70% active, supplier: P&G
3 Hexyl Cinnamic Aldehyde, supplier: International Flavors & Fragrances, Inc.
4 Sodium Chloride, supplier: Morton

| Ingredient | Examples, active wt% | |
|---|---|---|
| | 13 (control) | 14 |
| Water | q. s. | q. s. |
| Sodium Laureth-1 Sulfate (SLE1S)[1] | 24.00 | - |
| Sodium Decyl Sulfate [2] | - | 24.00 |
| Hexyl Cinnamic Aldehyde [3] (ClogP = 4.3) | 0.50 | 0.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 5.02E-11 | 1.64E-10 |
| Diffusion Coefficient of Surfactant-soluble agent, $D_A$ ($m^2/s$) | 4.62E-11 | 7.91E-11 |
| Ratio of Diffusion Coefficients, $D_S/D_A$ | 1.1 | 2.1 |
| | | |
| Average area per gram of Hexyl Cinnamic Aldehyde | 2.14E+04 | 4.46E+04 |
| Deposition Efficiency (vs control) | 1.0X | 2.1X |

1 Sodium Laureth-1 Sulfate at 26% active, supplier: P&G
2 Sodium Undecyl Sulfate at 70% active, supplier: P&G
3 Hexyl Cinnamic Aldehyde, supplier: International Flavors & Fragrances, Inc.
4 Sodium Chloride, supplier: Morton

| Ingredient | | Examples, active wt% |
|---|---|---|
| | 15 (control) | 16 |
| Water | q. s. | q. s. |
| Sodium Laureth-1 Sulfate (SLE1S)[1] | 24.00 | - |
| Sodium Decyl Sulfate [2] | - | 24.00 |
| Linalyl Acetate [3] (ClogP = 3.8) | 0.50 | 0.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 5.30E-11 | 1.59E-10 |
| Diffusion Coefficient of Surfactant-soluble agent, $D_A$ ($m^2/s$) | 5.25E-11 | 9.22E-11 |
| Ratio of Diffusion Coefficients, $D_S/D_A$ | 1.0 | 1.7 |
| | | |
| Average area per gram of Linalyl Acetate | 1.94E+06 | 2. 86E+06 |
| Deposition Efficiency (vs control) | 1.0X | 1. 5X |

1 Sodium Laureth-1 Sulfate at 26% active, supplier: P&G
2 Sodium Undecyl Sulfate at 70% active, supplier: P&G
3 Linalyl Acetate, supplier: Givaudan
4 Sodium Chloride, supplier: Morton

| Ingredient | Examples, active wt% | |
|---|---|---|
| | 17 (control) | 18 |
| Water | q. s. | q. s. |
| Sodium Laureth-1 Sulfate (SLE1S)[1] | 24.00 | - |
| Sodium Decyl Sulfate [2] | - | 24.00 |
| Helvetolide [3] (ClogP = 5.6) | 0.50 | 0.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 4.86E-11 | 1.61E-10 |
| Diffusion Coefficient of Surfactant-soluble agent, $D_A$ ($m^2/s$) | 4.56E-11 | 8.61E-11 |
| Ratio of Diffusion Coefficients, $D_S/D_A$ | 1.1 | 1.9 |
| | | |
| Average area per gram of Helvetolide | 2.37E+05 | 8.23E+05 |
| Deposition Efficiency (vs control) | 1.0X | 3.5X |
| 1 Sodium Laureth-1 Sulfate at 26% active, supplier: P&G<br>2 Sodium Undecyl Sulfate at 70% active, supplier: P&G<br>3 Helvetolide, supplier: Firmenich<br>4 Sodium Chloride, supplier: Morton | | |

| Ingredient | Examples, active wt% | |
|---|---|---|
| | 19 (control) | 20 |
| Water | q. s. | q. s. |
| Sodium Laureth-1 Sulfate (SLE1S)[1] | 24.00 | - |
| Sodium Decyl Sulfate [2] | - | 24.00 |
| Polysantol[3] (ClogP = 4.2) | 0.50 | 0.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 5.18E-11 | 1.67E-10 |
| Diffusion Coefficient of Surfactant-soluble agent, $D_A$ ($m^2/s$) | 4.78E-11 | 8.73E-11 |
| Ratio of Diffusion Coefficients, $D_S/D_A$ | 1.1 | 1.9 |
| | | |
| Average area per gram of Polysantol | 2.90E+05 | 9.01E+05 |
| Deposition Efficiency (vs control) | 1.0X | 3.1X |
| 1 Sodium Laureth-1 Sulfate at 26% active, supplier: P&G<br>2 Sodium Undecyl Sulfate at 70% active, supplier: P&G<br>3 Polysantol, supplier: Firmenich<br>4 Sodium Chloride, supplier: Morton | | |

| Ingredient | Examples, active wt% | |
|---|---|---|
| | 21 (control) | 22 (comparative example) |
| Water | q. s. | q. s. |
| Sodium Laureth-1 Sulfate (SLE1S)[1] | 24.00 | - |
| Sodium Decyl Sulfate [2] | - | 24.00 |
| Methyl Dihydrojasmonate[3] (ClogP = 3.0) | 0.50 | 0.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 5.12E-11 | 1.65E-10 |
| Diffusion Coefficient of Surfactant-soluble agent, $D_A$ ($m^2/s$) | 6.12E-11 | 8.27E-11 |
| Ratio of Diffusion Coefficients, $D_S/D_A$ | 0.84 | 2.0 |
| | | |
| Average area per gram of Methyl Dihydrojasmonate | 2. 66E+04 | 1.92E+04 |
| Deposition Efficiency (vs control) | 1.0X | 0.7X |

1 Sodium Laureth-1 Sulfate at 26% active, supplier: P&G
2 Sodium Undecyl Sulfate at 70% active, supplier: P&G
3 Methyl Dihydrojasmonate, supplier: Firmenich
4 Sodium Chloride, supplier: Morton

**Discussion of Results for Examples 11-22**

In Examples 11-20, the surfactant soluble agents have ClogP values greater than 3.0. Additionally, the Sodium Decyl Sulfate containing examples all have ratios of diffusion coefficients (DSIDA) greater than 1.2 which indicate that the respective surfactant soluble agents are diffusing at a different rate than the Sodium Decyl Sulfate micelles, which allows one to infer that the surfactant soluble agents are not within those micelles. In comparison, the Sodium Laureth-1 Sulfate containing examples (control) all have ratios of diffusion coefficients close to 1.0 which indicates that the surfactant soluble agents are diffusing at the same rate as the SLE1S micelles. This allows one to infer that the surfactant soluble agents are within the SLE1S micelles. Consequently, Examples 12, 14, 16, 18 and 20 are representative of the present invention and exhibit significantly greater deposition efficiency which is 1. 5X-4.7X that of their respective controls.

Example 22 is a comparative example and Example 21 is its control - both contain a surfactant soluble agent having a ClogP equal to 3.0. This set of examples demonstrates that if a surfactant soluble agent with ClogP less than or equal to 3.0 is used, then even if a diffusion coefficient ratio of greater than 1.2 is achieved, the deposition efficiency will be low and similar to the control (Example 21).

| Ingredient | Examples, active wt% | |
|---|---|---|
| | 23 (control) | 24 |
| Water | q. s. | q. s. |
| Sodium Laureth-1 Sulfate (SLE1S)[1] | 24.00 | - |
| Sodium Decyl Sulfate [2] | - | 24.00 |
| Deposition Accord [3] (average ClogP = 4.17) | 1.50 | 1.50 |
| Sodium Chloride [4] | Up to 2% | Up to 2% |
| Preservatives, pH adjusters | Up to 1% | Up to 1% |
| | | |
| Diffusion Coefficient of Surfactant, $D_S$ ($m^2/s$) | 4.97E-11 | 1.56E-10 |

(continued)

| Ingredient | Examples, active wt% | |
|---|---|---|
| | 23 (control) | 24 |
| Diffusion Coefficient of Surfactant-soluble agent, $D_A$ ($m^2/s$) - Deposition Accord | 4.72E-11 | 8.35E-11 |
| Ratio of Diffusion Coefficients, $D_S/D_A$- Deposition Accord | 1.1 | 1.9 |
| | | |
| Average area per gram of Deposition Accord | 1.35E+06 | 2.24E+06 |
| Deposition Accord Deposition Efficiency (vs control) | 1.0X | 1.7X |
| 1 Sodium Laureth-1 Sulfate at 26% active, supplier: P&G<br>2 Sodium Undecyl Sulfate at 70% active, supplier: P&G<br>3 Deposition Accord = 20% Hexyl Cinnamic Aldehyde + 20% Linalyl Acetate + 20% Helvetolide + 20% Methyl Dihydrojasmonate + 20% Polysantol<br>4 Sodium Chloride, supplier: Morton | | |

**Discussion of Results for Examples 23-24**

Five of the previously exemplified surfactant soluble agents are premixed on an equal weight basis to make the Deposition Accord. This accord is then solubilized into either Sodium Laureth-1 Sulfate (Example 23) or Sodium Decyl Sulfate (Example 24). This set of examples demonstrates that even when multiple surfactant soluble agents are combined, when the average ClogP is greater than 3.0 and the average diffusion coefficient ratio (DS/DA) is greater than 1.2, then the deposition efficiency of the mixture will still be significantly increased (1.7X) versus the control.

## Claims

**1.** A personal care composition comprising:

a. from 14% to 40% of one or more surfactants, by weight of the total composition, wherein the one or more surfactants is an anionic surfactant selected from the group consisting of:

a)

$$R_1\ O(CH_2CHR_3O)_y\ SO_3M;$$

b)

$$CH_3\ (CH_2)_z\ CHR_2\ CH_2\ O\ (CH_2\ CHR_3O)_y\ SO_3M;$$

and
c) mixtures thereof,
where $R_1$ represents $CH_3\ (CH_2)_{10}$, $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is 1 when y is not zero (0), and
M is a monovalent or divalent, positively-charged cation,
wherein the total amount of one or more surfactants ranges from 14% to 40% by weight of the total composition;

b. from 0.1% to 10% of one or more surfactant soluble agents having a ClogP greater than 3.0, by weight of the total composition, preferably greater than 3.2; preferably greater than 3.4, as determined by the fragment approach of Hansch & Leo, in Comprehensive Medicinal Chemistry, Vol. 4, Hansch, et al., Eds., p. 295, Pergamon Press, 1990,

wherein the one or more surfactant soluble agents comprises a hair health active, wherein the hair health

active is selected from the group consisting of isostearyl isostearate, histidine and mixtures thereof, wherein the total amount of one or more surfactant soluble agents having a ClogP greater than 3.0 ranges from 0.1% to 10% by weight of the total composition;
wherein when the personal care composition is diluted to 1.5% surfactant concentration, the personal care composition has a ratio of surfactant diffusion coefficient to soluble agent diffusion coefficient greater than 1.2, preferably greater than 1.4, preferably greater than 1.6.

**2.** A personal care composition according to claim 1 further comprising from 0.25% to 15% of one or more amphoteric, nonionic or zwitterionic co-surfactants, by weight of the total composition.

**3.** A personal care composition according to any preceding claims wherein the composition further comprises a cationic polymer.

**4.** A personal care composition according to any preceding claims wherein the composition further comprises a conditioning agent, preferably wherein the conditioning agent is a silicone.

**5.** A personal care composition according to any preceding claims further comprising one or more scalp health agent, preferably wherein the scalp health agent is selected from the group consisting of zinc pyrithione, selenium sulfide, menthol and/or menthyl lactate and mixtures thereof.

**6.** A foam dispenser comprising the personal care composition according to any preceding claims.

**7.** The foam dispenser of claim 6, wherein the foam dispenser is an aerosol foam dispenser.

**8.** The foam dispenser according to claim 6, wherein the foam dispenser is an pumped foam dispenser.

**Patentansprüche**

**1.** Körperpflegezusammensetzung, umfassend:

a. von zu 14 Gew.-% bis 40 Gew.-% der Gesamtzusammensetzung ein oder mehrere Tenside,
wobei das eine oder die mehreren Tenside ein anionisches Tensid ist, das aus der Gruppe ausgewählt ist, bestehend aus:

a)

$$R_1\ O(CH_2CHR_3O)_y\ SO_3M;$$

b)

$$CH_3\ (CH_2)_z\ CHR_2\ CH_2\ O\ (CH_2\ CHR_3O)_y\ SO_3M;$$

und
c) Mischungen davon,

wobei $R_1$ $CH_3$ $(CH_2)_{10}$ darstellt, $R_2$ H oder ein Kohlenwasserstoffradikal darstellt, umfassend 1 bis 4 Kohlenstoffatome derart, dass die Summe der Kohlenstoffatome in z und $R_2$ 8 ist, $R_3$ H oder $CH_3$ ist, y 0 bis 7 ist, der Durchschnittswert von y 1 ist, wenn y nicht null (0) ist, und M ein einwertiges oder zweiwertiges, positiv geladenes Kation ist,
wobei die Gesamtmenge eines oder mehrerer Tenside in einem Bereich von zu 14 Gew.-% bis 40 Gew.-% der Gesamtzusammensetzung liegt;

b. von zu 0,1 Gew.-% bis 10 Gew.-% ein oder mehrere tensidlösliche Mittel, die einen ClogP über 3,0 Gew.-% der Gesamtzusammensetzung, vorzugsweise über 3,2, aufweisen; vorzugsweise über 3,4, wie durch den Fragment-Ansatz von Hansch&Leo in Compenensive Medicinal Chemistry, Vol. 4, Hansch, et al., Hrsgg., S. 295, Pergamon Press, 1990, bestimmt,

wobei das eine oder die mehreren tensidlöslichen Mittel einen Haargesundheitswirkstoff umfassen, wobei der Haargesundheitswirkstoff aus der Gruppe ausgewählt ist, bestehend aus Isostearylisostearat, Histidin und Mischungen davon,
wobei die Gesamtmenge eines oder mehrerer tensidlöslicher Mittel, die einen ClogP über 3,0 aufweisen, in dem Bereich von zu 0,1 Gew.-% bis 10 Gew.-% der Gesamtzusammensetzung liegt;
wobei, wenn die Körperpflegezusammensetzung auf 1,5 % Tensidkonzentration verdünnt wird, die Körperpflegezusammensetzung ein Verhältnis von Tensiddiffusionskoeffizient zu Diffusionskoeffizient eines löslichen Mittels von über 1,2, vorzugsweise über 1,4, vorzugsweise über 1,6, aufweist.

**2.** Körperpflegezusammensetzung nach Anspruch 1, ferner umfassend von zu 0,25 Gew.-% bis 15 Gew.-% ein oder mehrere amphotere, nichtionische oder zwitterionische Cotenside der Gesamtzusammensetzung.

**3.** Körperpflegezusammensetzung nach einem der vorstehenden Ansprüche, wobei die Zusammensetzung ferner ein kationisches Polymer umfasst.

**4.** Körperpflegezusammensetzung nach einem der vorstehenden Ansprüche, wobei die Zusammensetzung ferner ein Konditioniermittel umfasst, vorzugsweise wobei das Konditioniermittel ein Silikon ist.

**5.** Körperpflegezusammensetzung nach einem der vorstehenden Ansprüche, ferner umfassend ein oder mehrere Kopfhautgesundheitsmittel, vorzugsweise wobei das Kopfhautgesundheitsmittel aus der Gruppe ausgewählt ist, bestehend aus Zink-Pyrithion, Selensulfid, Menthol und/oder Menthyllactat und Mischungen davon.

**6.** Schaumspender, umfassend die Körperpflegezusammensetzung nach einem der vorstehenden Ansprüche.

**7.** Schaumspender nach Anspruch 6, wobei der Schaumspender ein Aerosolschaumspender ist.

**8.** Schaumspender nach Anspruch 6, wobei der Schaumspender ein Pumpschaumspender ist.

## Revendications

**1.** Composition de soins personnels comprenant :

a. de 14 % à 40 % d'un ou plusieurs tensioactifs, en poids de la composition totale,
dans laquelle le ou les tensioactifs est un tensioactif anionique choisi dans le groupe constitué par :

a)

$$R_1\ O(CH_2CHR_3O)_y\ SO_3M\ ;$$

b)

$$CH_3\ (CH_2)_z\ CHR_2\ CH_2\ O\ (CH_2\ CHR_3O)_y\ SO_3M\ ;$$

et
c) leurs mélanges,

où $R_1$ représente $CH_3\ (CH_2)_{10}$, $R_2$ représente H ou un radical hydrocarboné comprenant 1 à 4 atomes de carbone de telle sorte que la somme des atomes de carbone dans z et $R_2$ vaut 8, $R_3$ est H ou $CH_3$, y va de 0 à 7, la valeur moyenne de y vaut 1 lorsque y n'est pas zéro (0), et M est un cation chargé positivement, monovalent ou divalent,
dans laquelle la quantité totale d'un ou plusieurs tensioactifs se situe dans une plage allant de 14 % à 40 %, en poids de la composition totale ;

b. de 0,1 % à 10 % d'un ou plusieurs agents solubles dans un tensioactif ayant un ClogP supérieur à 3,0, en poids de la composition totale, de préférence supérieur à 3,2 ; de préférence supérieur à 3,4, tel que déterminé par l'approche par fragments de Hansch & Leo, dans Comprehensive Medicinal Chemistry, vol. 4, Hansch, et al., Éd., 295, Pergamon Press, 1990,

dans laquelle le ou les agents solubles dans un tensioactif comprennent un actif pour la santé des cheveux, dans laquelle l'actif pour la santé des cheveux est choisi dans le groupe constitué par l'isostéarate d'isostéaryle, l'histidine et leurs mélanges,
dans laquelle la quantité totale d'un ou plusieurs agents solubles dans un tensioactif ayant un ClogP supérieur à 3,0 se situe dans une plage allant de 0,1 % à 10 % en poids de la composition totale ;
dans laquelle lorsque la composition de soins personnels est diluée à 1,5 % de concentration de tensioactif, la composition de soins personnels présente un rapport entre le coefficient de diffusion de tensioactif et le coefficient de diffusion d'agent soluble supérieur à 1,2, de préférence supérieur à 1,4, de préférence supérieur à 1,6.

**2.** Composition de soins personnels selon la revendication 1 comprenant en outre de 0,25 % à 15 % d'un ou plusieurs cotensioactifs amphotères, non ioniques ou zwittérioniques, en poids de la composition totale.

**3.** Composition de soins personnels selon de quelconques revendications précédentes dans laquelle la composition comprend en outre un polymère cationique.

**4.** Composition de soins personnels selon de quelconques revendications précédentes dans laquelle la composition comprend en outre un agent de conditionnement, de préférence dans laquelle l'agent de conditionnement est une silicone.

**5.** Composition de soins personnels selon de quelconques revendications précédentes comprenant en outre un ou plusieurs agents pour la santé du cuir chevelu, de préférence dans laquelle l'agent pour la santé du cuir chevelu est choisi dans le groupe constitué par la pyrithione de zinc, le sulfure de sélénium, le menthol et/ou le lactate de menthyle et leurs mélanges.

**6.** Distributeur de mousse comprenant la composition de soins personnels selon de quelconques revendications précédentes.

**7.** Distributeur de mousse selon la revendication 6, dans lequel le distributeur de mousse est un distributeur de mousse aérosol.

**8.** Distributeur de mousse selon la revendication 6, dans lequel le distributeur de mousse est un distributeur de mousse à pompe.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010141683 A2
- US 8440605 B
- US 2009155383
- US 20090221463
- US 5104646 A
- US 5106609 A
- US 3929678 A
- US 2658072 A
- US 2438091 A
- US 2528378 A
- WO 9406403 A, Reich
- US 34584 A
- US 6316541 B
- US 4476282 A
- US 20070276087
- US 5284972 A
- US 5747440 A
- WO 2004078903 A
- WO 2004078901 A
- WO 2005078063 A


### Non-patent literature cited in the description


- **PATIST, A. ; JHA, B.K. ; OH, S.G. ; SHAH, D.O.** *J. Surfactants Deterg.,* 1999, vol. 2, 317
- **JAMES-SMITH, M.A. ; SHEKHAWAT, D. ; SHAH, D.O.** *Tenside Surf. Det.,* 2007, vol. 44, 142
- **HANSCH ; LEO et al.** Comprehensive Medicinal Chemistry. Pergamon Press, 1990, 295
- **LEO et al.** Comprehensive Medicinal Chemistry. Pergamon Press, 1990, vol. 4, 295
- McCutcheion's Detergents and Emulsifiers. Allured Publishing Corp, 1986
- McCutcheion's Functional Materials. 1992
- McCutcheon's, Emulsifiers and Detergents. M. C. Publishing Co, 1989
- Cationic Starches. **SOLAREK, D. B.** Modified Starches: Properties and Uses. CRC Press, Inc, 1986, 113-125
- **QIN-JI PENG ; ARTHUR S. PERLIN.** Observation on NMR Spectra of Starches in Dimethyl Sulfoxide, Iodine-Complexing, and Solvating in Water-Dimethyl Sulfoxide. *Carbohydrate Research,* 1987, vol. 160, 57-72
- **J. HOWARD BRADBURY ; J. GRANT COLLINS.** An Approach to the Structural Analysis of Oligosaccharides by NMR Spectroscopy. *Carbohydrate Research,* 1979, vol. 71, 15-25
- **STEVEN ABBOTT ; HIROSHI YAMAMOTO.** HSPIP - Hansen Solubility Parameters in Practice
- CTFA Cosmetic Ingredient Handbook. Cosmetic, Toiletry, and Fragrance Association, Inc, 2004
- Encyclopedia of Polymer Science and Engineering,. John Wiley & Sons, Inc, 1989, vol. 15, 204-308
- **SMITH, A. L.** The Analytical Chemistry of Silicones. John Wiley & Sons, Inc, 1991
- **A E MARTELL ; R M SMITH.** Critical Stability Constants. Plenum Press, 1974, vol. 1
- **A E MARTELL ; R D HANCOCK.** Metal Complexes in Aqueous Solution. Plenum Press, 1996